# EUROPEAN PATENT APPLICATION

(11) **EP 3 521 435 A1**
(43) Date of publication of application: **07.08.2019**
(21) Application number: 18155393.4
(22) Date of filing: 06.02.2018
(51) Int. Cl.: C12N 15/67, C12N 15/79

(54) **A HIGH STRINGENCY SELECTION SYSTEM FOR PROTEIN EXPRESSION IN PROLINE-AUXOTROPHIC CELLS**

(71) Applicant: Bayer AG, 51373 Leverkusen (DE); Bayer Pharma Aktiengesellschaft, 13353 Berlin (DE)
(72) Inventor: TRAUTWEIN, Mark, 42489 Wülfrath (DE); KLEYMANN, Ulrike, 42119 Wuppertal (DE); BROEHLAND, Bettina, 42119 Wuppertal (DE)
(74) Representative: BIP Patents

(57) **Abstract**

The present invention relates to a high stringency metabolic selection system for protein expression in eukaryotic cells. In particular, it relates to use of compounds as well as media, vectors and methods for culturing proline-auxotrophic cells under conditions of high stringency for efficient screening of cells expressing a POI.

SEQUENCE LISTING

<110>  Bayer Aktiengesellschaft
       Bayer Pharma Aktiengesellschaft

<120>  A High Stringency Selection System for Protein Expression in
       Proline-auxotrophic Cells

<130>  BHC171058

<160>  6

<170>  PatentIn version 3.5

<210>  1
<211>  795
<212>  PRT
<213>  Mus musculus

<400>  1

Met Leu Arg His Met His Arg Ser Gly Val Gln Pro Phe Arg Gln Arg
1               5                   10                  15

Leu Leu Pro Trp Val Gln Ser Ile Ala Val Pro Arg Ser Asn Arg Val
            20                  25                  30

Gln Pro Ser Ala Ile Arg His Val Arg Ser Trp Ser Asn Ile Pro Phe
        35                  40                  45

Ile Thr Val Pro Leu Ser Arg Ala His Gly Lys Pro Phe Ala His Arg
    50                  55                  60

Ser Glu Leu Lys His Ala Lys Arg Ile Val Val Lys Leu Gly Ser Ala
65                  70                  75                  80

Val Val Thr Arg Gly Asp Glu Cys Gly Leu Ala Leu Gly Arg Leu Ala
                85                  90                  95

Ser Ile Val Glu Gln Val Ser Val Leu Gln Asn Gln Gly Arg Glu Met
            100                 105                 110

Met Leu Val Thr Ser Gly Ala Val Ala Phe Gly Lys Gln Arg Leu Arg
        115                 120                 125

His Glu Ile Leu Leu Ser Gln Ser Val Arg Gln Ala Leu His Ser Gly
    130                 135                 140

Gln Asn His Leu Lys Glu Met Ala Ile Pro Val Leu Glu Ala Arg Ala
145                 150                 155                 160

Cys Ala Ala Ala Gly Gln Ser Gly Leu Met Ala Leu Tyr Glu Ala Met
                165                 170                 175

Phe Thr Gln Tyr Ser Ile Cys Ala Ala Gln Ile Leu Val Thr Asn Leu
            180                 185                 190

Asp Phe His Asp Glu Gln Lys Arg Arg Asn Leu Asn Gly Thr Leu His
        195                 200                 205

Glu Leu Leu Arg Met Asn Ile Val Pro Ile Val Asn Thr Asn Asp Ala
    210                 215                 220

Val Val Pro Pro Ala Glu Pro Asn Ser Asp Leu Gln Gly Val Asn Val
225                 230                 235                 240

Ile Ser Val Lys Asp Asn Asp Ser Leu Ala Ala Arg Leu Ala Val Glu
                245                 250                 255

Met Lys Thr Asp Leu Leu Ile Val Leu Ser Asp Val Glu Gly Leu Phe
            260                 265                 270

Asp Ser Pro Pro Gly Ser Asp Asp Ala Lys Leu Ile Asp Ile Phe Tyr
        275                 280                 285

Pro Gly Asp Gln Gln Ser Val Thr Phe Gly Thr Lys Ser Arg Val Gly
    290                 295                 300

Leu Gly Gly Met Glu Ala Lys Val Lys Ala Ala Leu Trp Ala Leu Gln
305                 310                 315                 320

Gly Gly Thr Ser Val Val Ile Ala Asn Gly Thr His Pro Lys Val Ser
                325                 330                 335

Gly His Val Ile Thr Asp Ile Val Glu Gly Lys Lys Val Gly Thr Phe
            340                 345                 350

Phe Ser Glu Val Lys Pro Ala Gly Pro Thr Val Glu Gln Gln Gly Glu
        355                 360                 365

Met Ala Arg Ser Gly Gly Arg Met Leu Ala Thr Leu Glu Pro Glu Gln
    370                 375                 380

Arg Ala Glu Ile Ile Asn His Leu Ala Asp Leu Leu Thr Asp Gln Arg
385                 390                 395                 400

Glu Glu Ile Leu Leu Ala Asn Lys Lys Asp Leu Glu Glu Ala Glu Gly
                405                 410                 415

Arg Leu Ala Ser Pro Leu Leu Lys Arg Leu Ser Leu Ser Thr Ser Lys

            420                 425                 430

Leu Asn Ser Leu Ala Ile Gly Leu Arg Gln Ile Ala Ala Ser Ser Gln
        435                 440                 445

Glu Ser Val Gly Arg Val Leu Arg Arg Thr Arg Ile Ala Lys Asn Leu
    450                 455                 460

Glu Leu Glu Gln Val Thr Val Pro Ile Gly Val Leu Leu Val Ile Phe
465                 470                 475                 480

Glu Ser Arg Pro Asp Cys Leu Pro Gln Val Ala Ala Leu Ala Ile Ala
                485                 490                 495

Ser Gly Asn Gly Leu Leu Leu Lys Gly Gly Lys Glu Ala Ala His Ser
            500                 505                 510

Asn Arg Ile Leu His Leu Leu Thr Gln Glu Ala Leu Ser Ile His Gly
        515                 520                 525

Val Lys Glu Ala Ile Gln Leu Val Asn Thr Arg Glu Glu Val Glu Asp
    530                 535                 540

Leu Cys Arg Leu Asp Lys Ile Ile Asp Leu Ile Ile Pro Arg Gly Ser
545                 550                 555                 560

Ser Gln Leu Val Arg Asp Ile Gln Lys Ala Ala Lys Gly Ile Pro Val
                565                 570                 575

Met Gly His Ser Glu Gly Ile Cys His Met Tyr Val Asp Ser Glu Ala
            580                 585                 590

Ser Val Asp Lys Val Thr Arg Leu Val Arg Asp Ser Lys Cys Glu Tyr
        595                 600                 605

Pro Ala Ala Cys Asn Ala Leu Glu Thr Leu Leu Ile His Arg Asp Leu
    610                 615                 620

Leu Arg Thr Pro Leu Phe Asp Gln Ile Ile Asp Met Leu Arg Val Glu
625                 630                 635                 640

Gln Val Lys Ile His Ala Gly Pro Lys Phe Ala Ser Tyr Leu Thr Phe
                645                 650                 655

Ser Pro Ser Glu Val Lys Ser Leu Arg Thr Glu Tyr Gly Asp Leu Glu
            660                 665                 670

Val Cys Ile Glu Val Val Asp Ser Val Gln Glu Ala Ile Asp His Ile
        675                 680                 685

His Lys Tyr Gly Ser Ser His Thr Asp Val Ile Val Thr Glu Asn Glu
    690                 695                 700

Lys Thr Ala Glu Phe Phe Leu Gln His Val Asp Ser Ala Cys Val Phe
705                 710                 715                 720

Trp Asn Ala Ser Thr Arg Phe Ser Asp Gly Tyr Arg Phe Gly Leu Gly
                725                 730                 735

Ala Glu Val Gly Ile Ser Thr Ser Arg Ile His Ala Arg Gly Pro Val
            740                 745                 750

Gly Leu Glu Gly Leu Leu Thr Thr Lys Trp Leu Leu Arg Gly Gln Asp
        755                 760                 765

His Val Val Ser Asp Phe Ser Glu His Gly Ser Leu Lys Tyr Leu His
    770                 775                 780

Glu Asn Leu Pro Val Pro Gln Arg Asn Phe Ser
785                 790                 795

<210>  2
<211>  2385
<212>  DNA
<213>  Mus musculus

<400>  2
atgctgagac acatgcaccg ctctggggtc cagcccttca ggcaacgtct tctaccatgg      60

gtccagtcca tagctgtgcc cagatcaaat cgtgtccagc cctcagctat tagacatgtt     120

cgttcttgga gcaacatccc ctttatcacg gtacccctca gtcgtgccca cggcaagccc     180

tttgcccacc gaagtgagct gaagcacgcc aagagaattg tagtgaaact cggcagtgcc     240

gtggtgacca gaggagatga gtgtggcctg gcactgggac gcctggcatc tattgttgaa     300

caggtctcgg tgctgcagaa tcagggccga gagatgatgc tggtgaccag tggagccgtc     360

gccttcggca aacagcgcct gcgccatgag atccttctgt ctcagagcgt gcggcaagcc     420

ctgcactcag gacagaacca tctgaaggag atggcaattc cggtcttaga ggcccgagcc     480

tgtgcagctg ctggacagag tggactgatg gccttgtacg aggccatgtt tacacagtac     540

agcatctgtg ctgcccagat cttggtgacc aacctggatt tccacgacga gcagaagcgc     600

agaaatctca acggcaccct ccacgagctg ctccgcatga acatcgtccc catcgtcaac     660

acaaacgatg ccgtcgtccc tccagccgag cccaacagtg atcttcaggg ggtaaatgtg     720

attagtgtta aggataatga tagcctggct gcccggctgg ccgtggagat gaaaaccgac     780

ctcttgattg tcctctcgga cgtagaaggc ctctttgaca gccccccagg ctcagatgat 840

gcaaagctca ttgatatatt ctatccggga gatcagcagt ctgtgacatt tggaaccaag 900

tctagagtgg gcctaggggg catggaagcc aaggtgaaag ccgccctctg ggctttgcaa 960

ggcgggactt ctgtcgtcat tgccaacggg acccacccaa aggtgtctgg gcacgtcatc 1020

acagacatcg tggagggaaa gaaagttggc accttctttt cagaagtgaa gcctgctggt 1080

cctacagtgg agcagcaggg ggagatggct cgatctggtg ggagaatgct ggctacctta 1140

gagcctgaac agagagctga aattatcaat catctggctg acctgctgac ggaccagcgg 1200

gaagagatcc tgttagccaa caaaaaagat ttggaggagg cagagggaag acttgccagc 1260

cccctgctga agcgccttag cctctccacg tccaaactga acagcctggc catcgggctg 1320

cggcagatcg cagcctcctc acaagagagt gtaggccgcg tcctgcgccg gactcggatt 1380

gccaaaaacc tggagttaga acaagtgact gtcccaatag gtgttttact ggtgatcttt 1440

gagtctcgcc ctgactgtct accccaggtg gcagccttgg ctattgccag tggcaatggc 1500

ttgttgctca aaggtgggaa ggaggccgca cacagcaacc gcattcttca cctcctgacc 1560

caggaggccc tctccatcca cggagtcaag gaggccatac agctggtaaa caccagagaa 1620

gaggttgagg atctctgccg ccttgacaaa ataatagatc tgatcattcc ccgaggctcc 1680

tcccagctgg tcagagacat ccagaaagct gccaaaggga tcccagtgat gggccacagc 1740

gaaggcatct gccacatgta cgtggattcc gaagccagcg tggacaaggt cacccgactc 1800

gtcagagact ctaaatgtga atacccagcc gcctgtaatg ccctggagac gttgcttatc 1860

caccgagatc tgctgagaac acccttattt gaccagatca ttgacatgct ccgagtggaa 1920

caggtaaaaa ttcacgcagg ccccaagttt gcctcctacc tgaccttcag cccctcagaa 1980

gtgaagtcac tccggacgga gtacggggac ctggaagtgt gcattgaagt ggtggacagc 2040

gtccaggaag ccattgatca catccataag tacggcagct ctcacacaga tgtcatcgtc 2100

accgagaacg agaaaacagc ggagttcttc ctccagcatg tggacagcgc ctgtgtcttc 2160

tggaacgcca gtactcgctt ctctgatggc taccgctttg gactgggagc tgaagttgga 2220

atcagcacat ctcgaatcca cgcccgggga ccagtaggac ttgagggatt gctaactaca 2280

aagtggttgc ttcgaggcca agaccacgtg gtctcggact tctccgagca cggaagcctc 2340

aagtatcttc acgagaatct ccctgttccc cagagaaatt tcagc 2385

<210> 3
<211> 4521
<212> DNA
<213> Artificial Sequence

<220>
<223> murine P5CS long isoform expression cassette

<400> 3
tggaatgtgt gtcagttagg gtgtggaaag tccccaggct ccccagcagg cagaagtatg 60
caaagcatgc atctcaatta gtcagcaacc aggtgtggaa agtccccagg ctccccagca 120
ggcagaagta tgcaaagcat gcatctcaat tagtcagcaa ccatagtccc gcccctaact 180
ccgcccatcc cgcccctaac tccgcccagt tccgcccatt ctccgcccca tggctgacta 240
atttttttta tttatgcaga ggccgaggcc gcctcggcct ctgagctatt ccagaagtag 300
tgaggaggct tttttggagg cctaggcttt tgcaaaaagg ttgccggcca ccgctcagag 360
caccttccac catatgctga gacacatgca ccgctctggg gtccagccct tcaggcaacg 420
tcttctacca tgggtccagt ccatagctgt gcccagatca aatcgtgtcc agccctcagc 480
tattagacat gttcgttctt ggagcaacat cccctttatc acggtacccc tcagtcgtgc 540
ccacggcaag ccctttgccc accgaagtga gctgaagcac gccaagagaa ttgtagtgaa 600
actcggcagt gccgtggtga ccagaggaga tgagtgtggc ctggcactgg gacgcctggc 660
atctattgtt gaacaggtct cggtgctgca gaatcagggc cgagagatga tgctggtgac 720
cagtggagcc gtcgccttcg gcaaacagcg cctgcgccat gagatccttc tgtctcagag 780
cgtgcggcaa gccctgcact caggacagaa ccatctgaag gagatggcaa ttccggtctt 840
agaggcccga gcctgtgcag ctgctggaca gagtggactg atggccttgt acgaggccat 900
gtttacacag tacagcatct gtgctgccca gatcttggtg accaacctgg atttccacga 960
cgagcagaag cgcagaaatc tcaacggcac cctccacgag ctgctccgca tgaacatcgt 1020
ccccatcgtc aacacaaacg atgccgtcgt ccctccagcc gagcccaaca gtgatcttca 1080
gggggtaaat gtgattagtg ttaaggataa tgatagcctg gctgcccggc tggccgtgga 1140
gatgaaaacc gacctcttga ttgtcctctc ggacgtagaa ggcctctttg acagcccccc 1200
aggctcagat gatgcaaagc tcattgatat attctatccg ggagatcagc agtctgtgac 1260
atttggaacc aagtctagag tgggcctagg gggcatggaa gccaaggtga aagccgccct 1320
ctgggctttg caaggcggga cttctgtcgt cattgccaac gggacccacc caaaggtgtc 1380
tgggcacgtc atcacagaca tcgtggaggg aaagaaagtt ggcaccttct tttcagaagt 1440
gaagcctgct ggtcctacag tggagcagca gggggagatg gctcgatctg gtgggagaat 1500
gctggctacc ttagagcctg aacagagagc tgaaattatc aatcatctgg ctgacctgct 1560
gacggaccag cgggaagaga tcctgttagc caacaaaaaa gatttggagg aggcagaggg 1620
aagacttgcc agccccctgc tgaagcgcct tagcctctcc acgtccaaac tgaacagcct 1680
ggccatcggg ctgcggcaga tcgcagcctc ctcacaagag agtgtaggcc gcgtcctgcg 1740
ccggactcgg attgccaaaa acctggagtt agaacaagtg actgtcccaa taggtgtttt 1800
actggtgatc tttgagtctc gccctgactg tctaccccag gtggcagcct tggctattgc 1860

cagtggcaat ggcttgttgc tcaaaggtgg gaaggaggcc gcacacagca accgcattct 1920

tcacctcctg acccaggagg ccctctccat ccacggagtc aaggaggcca tacagctggt 1980

aaacaccaga gaagaggttg aggatctctg ccgccttgac aaaataatag atctgatcat 2040

tccccgaggc tcctcccagc tggtcagaga catccagaaa gctgccaaag ggatcccagt 2100

gatgggccac agcgaaggca tctgccacat gtacgtggat tccgaagcca gcgtggacaa 2160

ggtcacccga ctcgtcagag actctaaatg tgaataccca gccgcctgta atgccctgga 2220

gacgttgctt atccaccgag atctgctgag aacaccctta tttgaccaga tcattgacat 2280

gctccgagtg gaacaggtaa aaattcacgc aggccccaag tttgcctcct acctgacctt 2340

cagcccctca gaagtgaagt cactccggac ggagtacggg gacctggaag tgtgcattga 2400

agtggtggac agcgtccagg aagccattga tcacatccat aagtacggca gctctcacac 2460

agatgtcatc gtcaccgaga acgagaaaac agcggagttc ttcctccagc atgtggacag 2520

cgcctgtgtc ttctggaacg ccagtactcg cttctctgat ggctaccgct ttggactggg 2580

agctgaagtt ggaatcagca catctcgaat ccacgcccgg ggaccagtag gacttgaggg 2640

attgctaact acaaagtggt tgcttcgagg gcaagaccac gtggtctcgg acttctccga 2700

gcacggaagc ctcaagtatc ttcacgagaa tctccctgtt ccccagagaa atttcagctg 2760

agaggtgcca ggcagtcggg accgttgtgc gccgtctggc acatggaggc tactccagga 2820

tctccagaga gaacctgatc tcgtccctca cttcttggaa gggtctgtct tctggacagt 2880

tcacctatgc ttctggctct cctcagcgtt tccaacctta gctaatgttc acactccatt 2940

ttggccttct tgggtgtgtc tgtctccgta actggggttg aatctagggc cttgcacatg 3000

gtaggcaaat gctccactgc caggctgtct ccctggccct gcagtccagt cctaggaaga 3060

gaatcgctgt tggagacaga gaggcttggc ttttcccagt ctgttcacat cagaggtaga 3120

ccctaaggga cgcatctacc atcagagaca atggaaagtc ctggctgccg gaacgcctca 3180

gtgaggtaaa gtctcttggc gttttgactg ggaccagccc agccattctc agcctctcga 3240

catgtaattt catttctttt acgaaagctc gtcttaagga gggctaactc cttaaaacct 3300

ttaacatttt cattggaaac ggtgactgag acagactgac tgtatccact ggggagatga 3360

ccttggtgtg tctgtgaagt tatgggcgca catcctagac atctgcagtc tgtctccggc 3420

ctggggacac atggtacctt caaagtagtt ggaatctgag catcacgctg gagggatgaa 3480

tttcaggatg ttcctacaag tgggaggaag atctacaatc atgcttgacc tttttataca 3540

aattattttg acatcctatt ctcgacctcc aggacaaaga ctagagatta ttttaatact 3600

ttagattctc cccccccccc tttttgagaa ataaaattct tataaaagcc aaaaaaaaaa 3660

aaaaaggcgg ccgcagatct ttgtgaagga accttacttc tgtggtgtga cataattgga 3720

caaactacct acagagattt aaagctctaa ggtaaatata aaatttttaa gtgtataatg 3780

tgttaaacta ctgattctaa ttgtttgtgt attttagatt ccaacctatg gaactgatga 3840

atgggagcag tggtggaatg cctttaatga ggaaaacctg ttttgctcag aagaaatgcc 3900

atctagtgat gatgaggcta ctgctgactc tcaacattct actcctccaa aaaagaagag 3960

aaaggtagaa gaccccaagg actttccttc agaattgcta agttttttga gtcatgctgt 4020

gtttagtaat agaactcttg cttgctttgc tatttacacc acaaaggaaa aagctgcact 4080

gctatacaag aaaattatgg aaaaatattc tgtaaccttt ataagtaggc ataacagtta 4140

taatcataac atactgtttt ttcttactcc acacaggcat agagtgtctg ctattaataa 4200

ctatgctcaa aaattgtgta cctttagctt tttaatttgt aaaggggtta ataaggaata 4260

tttgatgtat agtgccttga ctagagatca taatcagcca taccacattt gtagaggttt 4320

tacttgcttt aaaaaacctc ccacacctcc ccctgaacct gaaacataaa atgaatgcaa 4380

ttgttgttgt taacttgttt attgcagctt ataatggtta caaataaagc aatagcatca 4440

caaatttcac aaataaagca tttttttcac tgcattctag ttgtggtttg tccaaactca 4500

tcaatgtatc ttatcatgtc t 4521

<210> 4
<211> 793
<212> PRT
<213> Mus musculus

<400> 4

Met Leu Arg His Met His Arg Ser Gly Val Gln Pro Phe Arg Gln Arg
1               5                   10                  15

Leu Leu Pro Trp Val Gln Ser Ile Ala Val Pro Arg Ser Asn Arg Val
            20                  25                  30

Gln Pro Ser Ala Ile Arg His Val Arg Ser Trp Ser Asn Ile Pro Phe
        35                  40                  45

Ile Thr Val Pro Leu Ser Arg Ala His Gly Lys Pro Phe Ala His Arg
    50                  55                  60

Ser Glu Leu Lys His Ala Lys Arg Ile Val Val Lys Leu Gly Ser Ala
65                  70                  75                  80

Val Val Thr Arg Gly Asp Glu Cys Gly Leu Ala Leu Gly Arg Leu Ala
                85                  90                  95

Ser Ile Val Glu Gln Val Ser Val Leu Gln Asn Gln Gly Arg Glu Met
            100                 105                 110

Met Leu Val Thr Ser Gly Ala Val Ala Phe Gly Lys Gln Arg Leu Arg
        115                 120                 125

His Glu Ile Leu Leu Ser Gln Ser Val Arg Gln Ala Leu His Ser Gly
    130                 135                 140

Gln Asn His Leu Lys Glu Met Ala Ile Pro Val Leu Glu Ala Arg Ala
145                 150                 155                 160

Cys Ala Ala Ala Gly Gln Ser Gly Leu Met Ala Leu Tyr Glu Ala Met
                165                 170                 175

Phe Thr Gln Tyr Ser Ile Cys Ala Ala Gln Ile Leu Val Thr Asn Leu
            180                 185                 190

Asp Phe His Asp Glu Gln Lys Arg Arg Asn Leu Asn Gly Thr Leu His
        195                 200                 205

Glu Leu Leu Arg Met Asn Ile Val Pro Ile Val Asn Thr Asn Asp Ala
    210                 215                 220

Val Val Pro Pro Ala Glu Pro Asn Ser Asp Leu Gln Gly Val Ile Ser
225                 230                 235                 240

Val Lys Asp Asn Asp Ser Leu Ala Ala Arg Leu Ala Val Glu Met Lys
                245                 250                 255

Thr Asp Leu Leu Ile Val Leu Ser Asp Val Glu Gly Leu Phe Asp Ser
            260                 265                 270

Pro Pro Gly Ser Asp Asp Ala Lys Leu Ile Asp Ile Phe Tyr Pro Gly
        275                 280                 285

Asp Gln Gln Ser Val Thr Phe Gly Thr Lys Ser Arg Val Gly Leu Gly
    290                 295                 300

Gly Met Glu Ala Lys Val Lys Ala Ala Leu Trp Ala Leu Gln Gly Gly
305                 310                 315                 320

Thr Ser Val Val Ile Ala Asn Gly Thr His Pro Lys Val Ser Gly His
                325                 330                 335

Val Ile Thr Asp Ile Val Glu Gly Lys Lys Val Gly Thr Phe Phe Ser
            340                 345                 350

Glu Val Lys Pro Ala Gly Pro Thr Val Glu Gln Gln Gly Glu Met Ala
        355                 360                 365

Arg Ser Gly Gly Arg Met Leu Ala Thr Leu Glu Pro Glu Gln Arg Ala
    370                 375                 380

Glu Ile Ile Asn His Leu Ala Asp Leu Leu Thr Asp Gln Arg Glu Glu
385                 390                 395                 400

Ile Leu Leu Ala Asn Lys Lys Asp Leu Glu Glu Ala Glu Gly Arg Leu
                405                 410                 415

Ala Ser Pro Leu Leu Lys Arg Leu Ser Leu Ser Thr Ser Lys Leu Asn
            420                 425                 430

Ser Leu Ala Ile Gly Leu Arg Gln Ile Ala Ala Ser Ser Gln Glu Ser
        435                 440                 445

Val Gly Arg Val Leu Arg Arg Thr Arg Ile Ala Lys Asn Leu Glu Leu
    450                 455                 460

Glu Gln Val Thr Val Pro Ile Gly Val Leu Leu Val Ile Phe Glu Ser
465                 470                 475                 480

Arg Pro Asp Cys Leu Pro Gln Val Ala Ala Leu Ala Ile Ala Ser Gly
                485                 490                 495

Asn Gly Leu Leu Leu Lys Gly Gly Lys Glu Ala Ala His Ser Asn Arg
            500                 505                 510

Ile Leu His Leu Leu Thr Gln Glu Ala Leu Ser Ile His Gly Val Lys
        515                 520                 525

Glu Ala Ile Gln Leu Val Asn Thr Arg Glu Glu Val Glu Asp Leu Cys
    530                 535                 540

Arg Leu Asp Lys Ile Ile Asp Leu Ile Ile Pro Arg Gly Ser Ser Gln
545                 550                 555                 560

Leu Val Arg Asp Ile Gln Lys Ala Ala Lys Gly Ile Pro Val Met Gly
                565                 570                 575

His Ser Glu Gly Ile Cys His Met Tyr Val Asp Ser Glu Ala Ser Val
            580                 585                 590

Asp Lys Val Thr Arg Leu Val Arg Asp Ser Lys Cys Glu Tyr Pro Ala
        595                 600                 605

Ala Cys Asn Ala Leu Glu Thr Leu Leu Ile His Arg Asp Leu Leu Arg

610                 615                 620

Thr Pro Leu Phe Asp Gln Ile Ile Asp Met Leu Arg Val Glu Gln Val
625                 630                 635                 640

Lys Ile His Ala Gly Pro Lys Phe Ala Ser Tyr Leu Thr Phe Ser Pro
                645                 650                 655

Ser Glu Val Lys Ser Leu Arg Thr Glu Tyr Gly Asp Leu Glu Val Cys
            660                 665                 670

Ile Glu Val Val Asp Ser Val Gln Glu Ala Ile Asp His Ile His Lys
        675                 680                 685

Tyr Gly Ser Ser His Thr Asp Val Ile Val Thr Glu Asn Glu Lys Thr
    690                 695                 700

Ala Glu Phe Phe Leu Gln His Val Asp Ser Ala Cys Val Phe Trp Asn
705                 710                 715                 720

Ala Ser Thr Arg Phe Ser Asp Gly Tyr Arg Phe Gly Leu Gly Ala Glu
                725                 730                 735

Val Gly Ile Ser Thr Ser Arg Ile His Ala Arg Gly Pro Val Gly Leu
            740                 745                 750

Glu Gly Leu Leu Thr Thr Lys Trp Leu Leu Arg Gly Gln Asp His Val
        755                 760                 765

Val Ser Asp Phe Ser Glu His Gly Ser Leu Lys Tyr Leu His Glu Asn
    770                 775                 780

Leu Pro Val Pro Gln Arg Asn Phe Ser
785                 790

<210>  5
<211>  2379
<212>  DNA
<213>  Mus musculus

<400>  5
   atgctgagac acatgcaccg ctctggggtc cagcccttca ggcaacgtct tctaccatgg      60
   gtccagtcca tagctgtgcc cagatcaaat cgtgtccagc cctcagctat tagacatgtt     120
   cgttcttgga gcaacatccc ctttatcacg gtacccctca gtcgtgccca cggcaagccc      180
   tttgcccacc gaagtgagct gaagcacgcc aagagaattg tagtgaaact cggcagtgcc     240
   gtggtgacca gaggagatga gtgtggcctg gcactgggac gcctggcatc tattgttgaa     300
   caggtctcgg tgctgcagaa tcagggccga gagatgatgc tggtgaccag tggagccgtc 360
   gccttcggca aacagcgcct gcgccatgag atccttctgt ctcagagcgt gcggcaagcc 420
   ctgcactcag gacagaacca tctgaaggag atggcaattc cggttttaga ggcccgagcc 480
   tgtgcagctg ctggacagag tggactgatg gccttgtacg aggccatgtt tacacagtac 540
   agcatctgtg ctgcccagat attggtgacc aacctggatt tccacgacga gcagaagcgc 600
   agaaatctca acggcaccct ccacgagctg ctccgcatga acatcgtccc catcgtcaac 660
   acaaacgatg ccgtcgtccc tccagccgag cccaacagtg atcttcaggg ggtgattagt 720
   gttaaggata atgatagcct ggctgcccgg ctggccgtgg agatgaaaac cgacctcttg 780
   attgtcctct cggacgtaga aggcctcttt gacagccccc caggctcaga tgatgcaaag 840
   ctcattgata tattctatcc gggagatcag cagtctgtga catttggaac caagtctaga 900
   gtgggcctag ggggcatgga agccaaggtg aaagccgccc tctgggcttt gcaaggcggg 960
   acttctgtcg tcattgccaa cgggacccac cccaaggtgt ctgggcacgt catcacagac 1020
   atcgtggagg gaaagaaagt tggcaccttc ttttcagaag tgaagcctgc tggtcctaca 1080
   gtggagcagc agggggagat ggctcgatct ggtgggagaa tgctggctac cttagagcct 1140
   gaacagagag ctgaaattat caatcatctg gctgacctgc tgacggacca gcgggaagag 1200
   atcctgttag ccaacaaaaa agatttggag gaggcagagg gaagacttgc cagccccctg 1260
   ctgaagcgcc ttagcctctc cacgtccaaa ctgaacagcc tggccatcgg gctgcggcag 1320
   atcgcagcct cctcacaaga gagtgtaggc cgcgtcctgc gccggactcg gattgccaaa 1380
   aacctggagt tagaacaagt gactgtccca ataggtgttt tactggtgat ctttgagtct 1440
   cgccctgact gtctacccca ggtggcagcc ttggctattg ccagtggcaa tggcttgttg 1500
   ctcaaaggtg ggaaggaggc cgcacacagc aaccgcattc ttcacctcct gacccaggag 1560
   gccctctcca tccacggagt caaggaggcc atacagctgg taaacaccag agaagaggtt 1620
   gaggatctct gccgccttga caaaataatc gatctgatca ttccccgagg ctcctcccag 1680
   ctggtcagag acatccagaa agctgccaaa gggatcccag tgatgggcca cagcgaaggc 1740
   atctgccaca tgtacgtgga ttccgaagcc agcgtggaca aggtcacccg actcgtcaga 1800
   gactctaaat gtgaataccc agccgcctgt aatgccctgg agacgttgct tatccaccga 1860
   gatttgctga gaacaccctt atttgaccag atcattgaca tgctccgagt ggaacaggta 1920
   aaaattcacg caggccccaa gtttgcctcc tacctgacct tcagcccctc agaagtgaag 1980
   tcactccgga cggagtacgg ggacctggaa gtgtgcattg aagtggtgga cagcgtccag 2040
   gaagccattg atcacatcca taagtacggc agctctcaca cagatgtcat cgtcaccgag 2100
   aacgagaaaa cagcggagtt cttcctccag catgtggaca gcgcctgtgt cttctggaac 2160
   gccagtactc gcttctctga tggctaccgc tttggactgg gagctgaagt tggaatcagc 2220
   acatctcgaa tccacgcccg gggaccagta ggacttgagg gattgctaac tacaaagtgg 2280
   ttgcttcgag ggcaagacca cgtggtctcg gacttctccg agcacggaag cctcaagtat 2340
   cttcacgaga atctccctgt tccccagaga aatttcagc 2379

<210> 6
<211> 3651
<212> DNA
<213> Artificial Sequence

<220>
<223> murine P5CS short isoform expression cassette

<400> 6
   tggaatgtgt gtcagttagg gtgtggaaag tccccaggct ccccagcagg cagaagtatg 60
   caaagcatgc atctcaatta gtcagcaacc aggtgtggaa agtccccagg ctccccagca 120
   ggcagaagta tgcaaagcat gcatctcaat tagtcagcaa ccatagtccc gcccctaact 180
   ccgcccatcc cgcccctaac tccgcccagt tccgcccatt ctccgcccca tggctgacta 240
   atttttttta tttatgcaga ggccgaggcc gcctcggcct ctgagctatt ccagaagtag 300
   tgaggaggct tttttggagg cctaggcttt tgcaaaaagg ttgccggcca ccgctcagag 360
   caccttccac catatgctga gacacatgca ccgctctggg gtccagccct tcaggcaacg 420
   tcttctacca tgggtccagt ccatagctgt gcccagatca aatcgtgtcc agccctcagc 480
   tattagacat gttcgttctt ggagcaacat cccctttatc acggtacccc tcagtcgtgc 540
   ccacggcaag ccctttgccc accgaagtga gctgaagcac gccaagagaa ttgtagtgaa 600
   actcggcagt gccgtggtga ccagaggaga tgagtgtggc ctggcactgg gacgcctggc 660
   atctattgtt gaacaggtct cggtgctgca gaatcagggc cgagagatga tgctggtgac 720
   cagtggagcc gtcgccttcg gcaaacagcg cctgcgccat gagatccttc tgtctcagag 780
   cgtgcggcaa gccctgcact caggacagaa ccatctgaag gagatggcaa ttccggtttt 840
   agaggcccga gcctgtgcag ctgctggaca gagtggactg atggccttgt acgaggccat 900
   gtttacacag tacagcatct gtgctgccca gatattggtg accaacctgg atttccacga 960
   cgagcagaag cgcagaaatc tcaacggcac cctccacgag ctgctccgca tgaacatcgt 1020
   ccccatcgtc aacacaaacg atgccgtcgt ccctccagcc gagcccaaca gtgatcttca 1080
   gggggtgatt agtgttaagg ataatgatag cctggctgcc cggctggccg tggagatgaa 1140
   aaccgacctc ttgattgtcc tctcggacgt agaaggcctc tttgacagcc ccccaggctc 1200
   agatgatgca aagctcattg atatattcta tccgggagat cagcagtctg tgacatttgg 1260
   aaccaagtct agagtgggcc tagggggcat ggaagccaag gtgaaagccg ccctctgggc 1320
   tttgcaaggc gggacttctg tcgtcattgc caacggaacc cacccaaagg tgtctgggca 1380
   cgtcatcaca gacatcgtgg agggaaagaa agttggcacc ttcttttcag aagtgaagcc 1440
   tgctggtcct acagtggagc agcaggggga gatggctcga tctggtggga gaatgctggc 1500
   taccttagag cctgaacaga gagctgaaat tatcaatcat ctggctgacc tgctgacgga 1560
   ccagcgggaa gagatcctgt tagccaacaa aaaagatttg gaggaggcag agggaagact 1620
   tgccagcccc ctgctgaagc gccttagcct ctccacgtcc aaactgaaca gcctggccat 1680
   cgggctgcgg cagatcgcag cctcctcaca agagagtgta ggccgcgtcc tgcgccggac 1740
   tcggattgcc aaaaacctgg agttagaaca agtgactgtc ccaataggtg ttttactggt 1800
   gatctttgag tctcgccctg actgtctacc ccaggtggca gccttggcta ttgccagtgg 1860
   caatggcttg ttgctcaaag gtgggaagga ggccgcacac agcaaccgca ttcttcacct 1920
   cctgacccag gaggccctct ccatccacgg agtcaaggag gccatacagc tggtaaacac 1980
   cagagaagag gttgaggatc tctgccgcct tgacaaaata atcgatctga tcattccccg 2040
   aggctcctcc cagctggtca gagacatcca gaaagctgcc aaaggatccc cagtgatggg 2100
   ccacagcgaa ggcatctgcc acatgtacgt ggattccgaa gccagcgtgg acaaggtcac 2160
   ccgactcgtc agagactcta aatgtgaata cccagccgcc tgtaatgccc tggagacgtt 2220
   gcttatccac cgagatttgc tgagaacacc cttatttgac cagatcattg acatgctccg 2280
   agtggaacag gtaaaaattc acgcaggccc caagtttgcc tcctacctga ccttcagccc 2340
   ctcagaagtg aagtcactcc ggacggagta cggggacctg gaagtgtgca ttgaagtggt 2400
   ggacagcgtc caggaagcca ttgatcacat ccataagtac ggcagctctc acacagatgt 2460
   catcgtcacc gagaacgaga aaacagcgga gttcttcctc cagcatgtgg acagcgcctg 2520
   tgtcttctgg aacgccagta ctcgcttctc tgatggctac cgctttggac tgggagctga 2580
   agttggaatc agcacatctc gaatccacgc ccggggacca gtaggacttg agggattgct 2640
   aactacaaag tggttgcttc gagggcaaga ccacgtggtc tcggacttct ccgagcacgg 2700
   aagcctcaag tatcttcacg agaatctccc tgttccccag agaaatttca gctaattaga 2760
   ctttgagtga tcttgagcct ttcctagttc atcccacccc gcccagatct ttgtgaagga 2820
   accttacttc tgtggtgtga cataattgga caaactacct acagagattt aaagctctaa 2880
   ggtaaatata aaatttttaa gtgtataatg tgttaaacta ctgattctaa ttgtttgtgt 2940
   attttagatt ccaacctatg gaactgatga atgggagcag tggtggaatg cctttaatga 3000
   ggaaaacctg ttttgctcag aagaaatgcc atctagtgat gatgaggcta ctgctgactc 3060
   tcaacattct actcctccaa aaaagaagag aaaggtagaa gaccccaagg actttccttc 3120
   agaattgcta agttttttga gtcatgctgt gtttagtaat agaactcttg cttgctttgc 3180
   tatttacacc acaaaggaaa aagctgcact gctatacaag aaaattatgg aaaaatattc 3240
   tgtaaccttt ataagtaggc ataacagtta taatcataac atactgtttt ttcttactcc 3300
   acacaggcat agagtgtctg ctattaataa ctatgctcaa aaattgtgta cctttagctt 3360
   tttaatttgt aaaggggtta ataaggaata tttgatgtat agtgccttga ctagagatca 3420
   taatcagcca taccacattt gtagaggttt tacttgcttt aaaaaacctc ccacacctcc 3480
   ccctgaacct gaaacataaa atgaatgcaa ttgttgttgt taacttgttt attgcagctt 3540
   ataatggtta caaataaagc aatagcatca caaatttcac aaataaagca tttttttcac 3600
   tgcattctag ttgtggtttg tccaaactca tcaatgtatc ttatcatgtc t 3651

## Description

### FIELD OF THE DISCLOSURE

The present invention relates to a high stringency metabolic selection system for protein expression in eukaryotic cells. In particular, it relates to use of compounds as well as media, vectors and methods for culturing proline-auxotrophic cells under conditions of high stringency for efficient screening of cells expressing a POI.

### BACKGROUND

In the early 1980s, the first recombinant insulin has been approved for therapeutic purposes. Since then, the biopharmaceutical industry has been rapidly growing. Currently, more than 100 recombinant protein therapeutics have been approved by US FDA or European Medicine Agency. Thus, there is a great need of the industry to support development of innovative biologics and biosimilars with more effective and efficient manufacturing processes using highly productive expression systems. More than half of the therapeutic proteins approved and marketed today are currently produced in Chinese Hamster Ovary (CHO) cell lines. Mammalian expression systems such as CHO cell lines can enable appropriate post-translational modification of the produced proteins, thereby facilitating large scale manufacturing processes.

Typically, an expression vector driving the expression of a protein of interest (POI) is introduced into a CHO host cell line or another production cell line. The aim is then to identify a clonal cell line where the expression vector has been successfully introduced and where the cell line is therefore producing the POI in a high amount with a desired product quality profile. However, successful introduction of the vector by stable integration into the genome is only a rare event, which is why the expression vector usually carries an additional selective marker to identify successfully transfected cell lines. Such a selective marker can be based on antibiotic selection or it can be a metabolic selection marker. Upon exposure of transfected cells to selective growth conditions, cells will typically only be able to survive if integration of at least the selective marker gene has occurred.

A variety of selection markers and systems for selecting transfected cells exist, including resistance to antibiotics such as neomycin, hygromycin, blasticidin, zeocin and Puromycin. Systems using an antibiotic selection marker have however several general drawbacks. The presence of an antibiotic during seed expansion or at least during the final stages of large scale production in a bioreactor requires additional caution with respect to patient safety requirements. Therefore, selective pressure preventing loss or silencing of expression of genes of interest cannot be maintained in final stages of production, often leading to loss of productivity.

With respect to metabolic selection markers, the most popular genes for selection are probably the genes encoding dihydrofolate reductase (DHFR), an enzyme involved in nucleotide metabolism (Haynes J, Weissmann C (1983) Nucleic Acid Res. 11(3):687-706), and glutamine synthetase (GS) (Cockett M et al (1990) Biotechnology NY 1990; 8:662-667, EP1196566). In both cases, selection occurs in the absence of the appropriate metabolite in the cell culture medium (hypoxantine and thymidine in the case of DHFR, glutamine in the case of GS), preventing growth of non-transformed cells. Both metabolic selection systems can be modulated in their selective stringency by using the presence of chemical inhibitors of DHFR (methotrexate, MTX) or GS (methionine sulfoximine, MSX). Of note, a major drawback of these and other chemical inhibitors is their (cyto)toxicity, as well as potential mutagenicity.

Expression of the marker gene is a necessary but not sufficient condition for obtaining a high producing cell line. Integration into the genome is a rare and random event and will yield different cell lines with highly different growth and productivity profiles. In addition, production stability over time of a certain cell line can be compromised. After extended cultivation, integrated genes may get lost or silenced in certain cell lines, particularly if cultivation under non-selective conditions is employed. In consequence, this results in a very time consuming and laborious screening process for obtaining a highly producing cell line suitable for manufacturing.

One option to optimize the selection process is an increase in stringency. In high stringency selection systems not only untransfected cells are eliminated but also cells with low expression of the selective marker. In consequence, more stringent selection systems usually create fewer colonies after transfection, but these colonies are likewise required to display on average higher expression values for a POI. High stringency selection leading to this increase in protein expression performance can then be used to reduce time consumption and labor associated with the screening process for the detection of colonies with high productivity. Van Blokland et al. describe a high stringency selection system based on antibiotic selection marker zeocin (Van Blokland et al., Cytotechnology. 2011 Aug;63(4):371-84; EP2443239). For instance, functionally impaired zeocin selection proteins were used for selection, resulting in a higher stringency. Similarly, lower levels of the zeocin selection marker protein were achieved by inefficient translation of the mRNA. While both variants resulted in a decreased number of stably transfected cell lines, these stably transfected cell lines displayed higher protein expression levels of the POI. A high stringency selection system based on a mutated folate receptor as selectable marker has been described in patent application US20160177318. Using folate receptor mutants with a decreased folate binding affinity as selection marker and selecting cells in medium with limited concentration of folic acid resulted in an increased stringency of selection when compared with either the DHFR/MTX system or with wild-type folate receptor as selection marker. In addition, combination of folate receptor with further selection markers, such as DHFR is suggested in patent application US20160177318. Using DHFR-negative CHO cell lines like CHO-DG44 or CHO-DXB11 is industry standard in conjunction with DHFR/MTX to overcome limitations in using cell lines like CHOK1, CHOK1SV or CHO-S, which still express DHFR endogenously. Therefore, DHFR/MTX is not compatible with every CHO expression platform used in industry. The same holds true, if folate receptor or its mutants are used as selection markers. The advantages of using a high stringency selection system based on GS have been reviewed by Fan et al. (Fan et al., Pharm. Bioprocess. 2013; 1(5):487-502). However, CHO cells express GS endogenously. Although this can be partially counteracted by the use of the GS inhibitor MSX, using a targeted CHO GS knockout cell was found to be superior. A major drawback of the GS-based system therefore lies in the necessity to generate a targeted GS knockout. Within a commercial setting, this is a lengthy and costly process. In addition, implementation into an existing CHO production platform with different CHO hosts or pre-existing different engineered CHO cell lines is complicated, since targeted knockouts would have to be created in multiple CHO hosts, and individual characterization would be necessary in every case.

Theoretically, the above mentioned disadvantages can be overcome by using a metabolic selection marker exploiting a natural auxotrophy that is present in all CHO cell lines in use. The term "CHO cell" comprises a large number of very different cell lines, all derived from the initial cloning of the original cell line from a spontaneously immortalized population of fibroblast cells from cultured ovarian cells of a partially inbred Chinese hamster. It is assumed that CHO cells have a clonal origin which is why the first CHO cells and all subsequently derived cell lines are deficient in proline synthesis (Wurm and Hacker, Nat Biotechnol. 2011 Aug 5;29(8):718-20). It has been shown that CHO cell lines are auxotrophic for proline due to a deficiency in both available proline catabolic pathways (Valle et al., Biochem Biophys Res Commun. 1973 Aug 21;53(4):1130-6; Baich, Somatic Cell Genet. 1977 Sep;3(5):529-38; Smith et al., Proc Natl Acad Sci USA. 1980 Sep;77(9):5221-5).

A key enzyme in this context is pyrroline-5-carboxylate synthase (P5CS). P5CS is an enzyme belonging to the family of oxidoreductases and participates in glutamate, arginine and proline metabolism. Heterologous expression of murine P5CS in CHO cells is sufficient to confer proline prototrophy and complement the growth phenotype in proline-free medium (Hu et al., J Biol Chem. 1999 Mar 5;274(10):6754-62). Moreover, Hu et al. have shown that murine P5CS can be used as a selective marker to select stably transfected CHO cells. In the same work, it was also shown that the two isoforms of murine P5CS are different with regards to feedback inhibition of P5CS activity by L-Ornithine. Both murine isoforms of P5CS, the long and the short isoform, complement proline auxotrophy in CHO cells. Whereas the short isoform is inhibited by L-Ornithine with a Kᵢ of approximately 0.25 mM, the long isoform is insensitive to Ornithine inhibition. However this approach to increase stringency does not result in a system which is superior to conventional antibiotic selection systems with respect to protein production. (EP2700713, US20140073007; Dissertation Anne Schüler "Development of a novel expression system for the production of recombinant proteins in Chinese hamster ovary cells based on the selection with a metabolic enzyme" Technische Universität Carolo-Wilhelmina zu Braunschweig, Germany, 2013). Patent application EP2700713 claims a polynucleotide and corresponding methods for screening and enriching a recombinant protein expressed in a eukaryotic cell, the polynucleotide comprising a tricistronic expression cassette comprising a) a promoter b) a gene of interest (GOI), c) a reporter gene, d) a selection marker gene, e) an internal ribosome entry site (IRES) element f) a 2A element. The approach to differentiate between the expression levels of gene of interest and selection marker is then to pose the IRES element downstream of the gene of interest and upstream of the selection marker. However, employing such a metabolic selection system based on P5CS and proline-free medium in CHO cells was found to be only just comparable to antibiotic selection with respect to protein expression, but clearly not superior to selection using an antibiotic marker. Therefore, there is the need for improvement and for overcoming this limitation of current P5CS-based selection, as this should be readily employable to all auxotrophic CHO host cell lines, thereby greatly facilitating screening efforts and improving on productivities without the additional complexities in manufacturing when using an antibiotic selection system. In particular, it is the object of the present invention to provide a high stringency selection system for selecting host cells producing a polypeptide of interest with high yield, as well as suitable expression vectors and host cells.

### SUMMARY

The current invention comprises tools and methods to allow for a high stringency P5CS based selection of proline-auxotrophic cells for efficient protein production. The method circumvents safety challenges known e.g. from antibiotic based systems, keeps the screening effort manageable and enables high production levels of a POI. Compared to conventional P5CS based selection systems, the stringency is increased, and also protein production is superior not only with regard to P5CS based selection systems but also relative to state of the art antibiotic selection systems. According to a first aspect, the current invention comprises the use of 3,4-dehydroproline (DHP) and/or at least one of its salts in a metabolic selection system, wherein the metabolic selective marker is a functional pyrroline-5-carboxylate synthase (P5CS). According to a second aspect, the current invention comprises a method for culturing and screening eukaryotic cells for the expression of high levels of one or more POI, the method comprising (i) introducing one or more polynucleotide(s) into a eukaryotic cell, wherein the one or more polynucleotide(s) encode(s) a functional P5CS and at least one POI, (ii) culturing the cell in an essentially proline-free cell culture medium, and (iii) culturing the cell in the presence of DHP and/or at least one of its salts. According to a third aspect of the current invention, there is provided a polynucleotide comprising a nucleic acid sequence encoding a functional P5CS and at least one POI, wherein the expression of the functional P5CS and the expression of at least one POI are under the control of different promoters, and wherein the promoter controlling said functional P5CS is weaker than the promoter controlling said at least one POI, and/or wherein the promoter controlling the expression of the functional P5CS is a weak promoter, such as a SV40 promoter, a truncated SV40 promoter or a Delta3SV40E promoter. According to a fourth aspect, the current invention comprises a method for culturing and screening eukaryotic cells for the expression of high levels of at least one POI, the method comprising a) introducing a polynucleotide according to the third aspect of the invention into a eukaryotic cell, and b) culturing the cell in an essentially proline-free cell culture medium. According to a fifth aspect, the current invention comprises an essentially proline-free cell culture medium comprising DHP and/or at least one of its salts for culturing and screening eukaryotic cells. According to a sixth aspect of the current invention, there is provided a eukaryotic cell, wherein at least one polynucleotide according to the third aspect has been introduced.

### BRIEF DESCRIPTION OF THE DRAWINGS

**Fig. 1****:** Schematic representation for selected readouts to characterize a selection system with respect to stringency.
**Fig. 2****:** Schematic representation of a suitable vector. Arrows represent regulatory elements. LC: light chain, HC: heavy chain. Open circles represent polyadenylation signals.
**Fig. 3****:** Growth of CHO-S cells in proline-free CD CHO medium. CHO-S host cell line fail to grow in chemically-defined proline-free medium, but growth can be rescued by re-addition of proline.
**Fig. 4****:** Viability of CHO-S cells in proline-free CD CHO medium. CHO-S host cell line fail to grow in chemically-defined proline-free medium, but growth can be rescued by re-addition of proline.

### BRIEF DESCRIPTION OF THE SEQUENCES

**SEQ ID NO:1** comprises murine P5CS long isoform amino acid sequence
**SEQ ID NO:2** comprises murine P5CS long isoform nucleotide sequence
**SEQ ID NO:3** comprises murine P5CS long isoform expression cassette nucleotide sequence
**SEQ ID NO:4** comprises murine P5CS short isoform amino acid sequence
**SEQ ID NO:5** comprises murine P5CS short isoform nucleotide sequence
**SEQ ID NO:6** comprises murine P5CS short isoform expression cassette nucleotide sequence

### DETAILED DESCRIPTION

### Definitions

Unless otherwise defined, all scientific and technical terms used in the description, figures and claims have their ordinary meaning as commonly understood by one of ordinary skill in the art. All publications, patent applications, patents, and other references mentioned herein are incorporated by reference in their entirety. In case of conflict, the present specification, including definitions, will prevail. If two or more documents incorporated by reference include conflicting and/or inconsistent disclosure with respect to each other, then the document having the later effective date shall control. The materials, methods, and examples are illustrative only and not intended to be limiting.

Unless otherwise stated, the following terms used in this document, including the description and claims, have the definitions given below.

The word **"about"** as used herein refers to a value being within an acceptable error range for the particular value as determined by one of ordinary skill in the art, which will depend in part on how the value is measured or determined, i.e., on the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects as described herein. In this context "about" may refer to a range above and/or below of up to 10%. Wherever the term "about" is specified for a certain assay or embodiment, that definition prevails for the particular context.

The terms **"comprising"**, **"including"**, **"containing"**, **"having"** etc. shall be read expansively or open-ended and without limitation. Singular forms such as **"a"**, **"an"** or **"the"** include plural references unless the context clearly indicates otherwise. Thus, for example, reference to a "cell" includes a single cell as well as a plurality of cells, either the same or different.

Unless otherwise indicated, the term **"at least"** preceding a series of elements is to be understood to refer to every element in the series. The terms **"at least one"** and **"at least one of"** include for example, one, two, three, four, five or more elements. It is furthermore understood that slight variations above and below a stated range can be used to achieve substantially the same results as a value within the range. Also, unless indicated otherwise, the disclosure of ranges is intended as a continuous range including every value between the minimum and maximum values.

The terms **"selective marker"**, **"selectable marker"** and **"selection marker"** are used interchangeable. A **"metabolic selective marker"** according to the current invention is a biomolecule such as a protein which by its presence or absence, enables or disables a certain metabolic pathway of the cell, thereby overcoming an auxotrophy of the cell, or leading to any other type of distinguishability of the populations. Typically, a metabolic selective marker can compensate for a corresponding metabolic or catabolic defect. A **"selective marker gene"** according to the current invention is a polynucleotide comprising a sequence encoding a selective marker protein.

The term **"3,4-dehydroproline" (DHP)** according to the current invention refers to the amino acid with the empirical formula (Hill Notation): C₅H₇NO₂ (CAS Number 4043-88-3) and/or its salts, such as HCl salt. Synonyms comprise 3,4-Dehydro-L-Proline, (S)-3-pyrroline-2-carboxylic acid, 3,4-didehydro-L-proline, 3,4-didehydroproline and 3,4-dehydro-L-pro-OH.

The term **"P5CS"** refers to the protein Delta-1-pyrroline-5-carboxylate synthase. The P5CS protein is encoded by the gene ALDH18A1. Alternative names for P5CS comprise aldehyde dehydrogenase family 18 member A1. The P5CS protein comprises human, murine, bovine, pig, dog, cat, Chinese hamster and further mammalian and non-mammalian homologues. The P5CS protein also comprises various plant homologues, such as rice or Arabidopsis homologues, as well as homologues from multiple further species. Sequence(s) for human P5CS are accessible via UniProt Identifier P54886 (P5CS_HUMAN), for instance human long isoform P54886-1 and human short isoform P54886-2. Sequence(s) for murine P5CS are accessible via UniProt Identifier Q9Z110 (P5CS_MOUSE), for instance murine long isoform Q9Z110-1 and murine short isoform Q9Z110-2. Sequence(s) for Chinese hamster P5CS are accessible for instance via UniProt Identifier G3HXX8-1 or A0A061IBJ4-1 (H671_3g8921). Different isoforms and variants may exist for the different species and are all comprised by the term P5CS. Also comprised are P5CS molecules before and after maturation, i.e., independent of cleavage of one or more pro-domains. In addition, synthetic variants of the P5CS protein may be generated and are comprised by the term P5CS. The protein P5CS may furthermore be subject to various modifications, e.g, synthetic or naturally occurring modifications. Examples for modifications are known in the art and include for example phosphorylation, glycosylation and lipidation. Recombinant P5CS is commercially available or can be manufactured as known in the art.

A **"functional pyrroline-5-carboxylate synthase"** according to the current invention is P5CS or another biomolecule catalyzing the reaction catalyzed by P5CS. In particular P5CS converts glutamate to glutamate 5-semialdehyde, an intermediate in the biosynthesis of proline, ornithine and arginine. The set of functional P5CS comprises for instance engineered or mutated versions of P5CS.

**A "protein of interest" (POI)** according to the current invention can be any linear chain of amino acid residues, with no further restriction applying as to the number or sequence of the amino acids.

The POI may furthermore be subject to various modifications, e.g, synthetic or naturally occurring modifications. Examples for modifications are known in the art and include for example phosphorylation, glycosylation and lipidation. A POI is encoded by a corresponding **"gene of interest" (GOI).**

The term **"antibody"** includes, but is not limited to, an immunoglobulin (e.g., IgG1, IgG2, IgG2a, IgG2b, IgG3, IgG4, IgM, IgD, IgE, IgA1, IgA2, IgA) and an antigen binding fragment thereof, but it also includes any proteinaceous binding molecule with immunoglobulin-like function. An antibody fragment generally contains an antigen binding or variable region. Examples of (recombinant) antibody fragments are immunoglobulin fragments such as Fab fragments, Fab' fragments, Fv fragments, single-chain Fv fragments (scFv), diabodies or domain antibodies (Holt, L.J., et al., Trends Biotechnol. (2003), 21, 11, 484-490).

The term **"auxotrophy"** refers to the inability of a cell or organism to synthesize a particular organic compound required for its growth. A **"proline auxotrophic"** cell according to the current invention is a cell which is deficient in proline synthesis.

The term **"cell culture medium"** refers to a nutrient solution or matrix used for culturing and/or selection of cells. A cell culture medium is typically adapted for the specific needs of the cultured cells. A multitude of compositions is commercially available or described in the literature. Cell culture media according to the current invention for eukaryotic cells can be or can be based on commercially available media such as Ham's F10, MEM, or DMEM. A cell culture medium is based on a second cell culture medium, if it comprises at least the majority of the ingredients of the second medium at the respective concentration +/- 20 %. Cell culture media typically comprise a carbon source. Cell culture media may comprise one or more further supplements such as amino acids, vitamins, salts or balanced salt solution, proteins such as growth factors, hormones, buffers, nucleosides, antibiotics, trace elements, lipids, sugars like mannose, glucose or glutamine, serum, pH indicator(s) or pH buffer components, or various others compounds. Cell culture media may contribute to the regulation of the physio-chemical environment of a cell (e.g. with respect to pH or osmotic pressure). Where a cell culture medium is used in a metabolic selection system, wherein the selection marker compensates for a corresponding metabolic or catabolic defect, the corresponding cell culture medium is required to be essentially free of the one or more metabolites the metabolic selection marker compensates for.

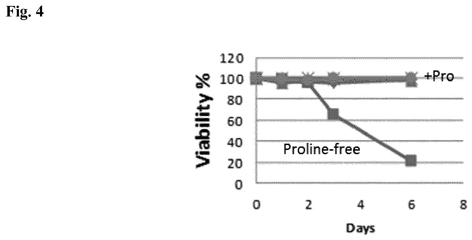

According to the current invention, a cell culture medium is called an **"essentially proline-free cell culture medium"** with respect to a certain cell line if it does not contain proline. A cell culture medium is also called an essentially proline-free cell culture medium with respect to a certain proline-auxotrophic cell line, if under standard conditions for culturing, the amount of proline in the cell culture medium is not sufficient to enable the survival of the majority of cells of that cell line (viability of 50 %) for at least six days. Viability can be determined as described in Example 13 and Fig. 4. Of note, it is possible that a cell culture medium is called essentially proline-free for a certain proline auxotrophic cell line and is called not essentially proline-free for another. In agreement with the given definition, a cell culture medium may still be called essentially proline-free, even if cultured cells have secreted minor amounts of proline into the medium after contacting the medium with the cells, if these minor amounts are not sufficient to enable the survival of the majority of cells of that cell line for at least six days (viability of 50 %).

The term **"polynuclotide"** refers to a recombinantly or synthetically produced polymeric desoxyribonucleotide or analog thereof, or a modified polynucleotide. The term comprises double and single stranded DNA or RNA. The polynucleotide can be integrated e.g. into minicircles, plasmids, cosmids, minichromosomes, or artificial chromosomes. The polynucleotide can be isolated or integrated in another nucleic acid molecule, e.g. in an expression vector or chromosome of a eukaryotic host cell.

**"Introducing a polynucleotide"** into a cell according to the current invention can be accomplished by transfection, transduction, transformation or any other technique that is suitable to transfer the polynucleotide into a cell. Genetical engineering of cells with routine molecular biology techniques for uptake and/or expression of a vector is well known in the art. It is also known in the art, that such an expression can be transient or stable. Techniques for transfection or viral transduction are described for instance in Sambrook et al. Molecular Cloning: A Laboratory Manual, Third Ed., Cold Spring Harbor Laboratory, New York 2001 and Ausubel et al., Curent Protocols in Molecular Biology, John Wiley and Sons, Baltimore, Md. (1999). Transfection of a polynucleotide or expression vector into a host cell can be carried out using standard techniques such as electroporation, nucleofection, calcium-phosphate precipitation, lipofection, polycation-based transfection such as polyethlylenimine (PEI)-based transfection, and DEAE-dextran transfection. Virus mediated gene delivery or Crispr/Cas based targeting methods are further options for introducing a polynucleotide into a cell according to the current invention.

The term **"vector",** as used herein, refers to a nucleic acid molecule capable of propagating a nucleic acid molecule to which it is linked. The term further comprises plasmids (non-viral) and viral vectors. Certain vectors are capable of directing the expression of nucleic acids or polynucleotides to which they are operatively linked. Such vectors are referred to herein as **"expression vectors".** Expression vectors for eukaryotic use can be constructed by inserting a polynucleotide sequence encoding at least one POI into a suitable vector backbone. The vector backbone can comprise the necessary elements to ensure maintenance of the vector and, if desirable, to provide amplification within the host. For viral vectors, e.g. lentiviral or retroviral vectors, further virus specific elements such as structural elements or other elements can be required and are well known in the art. These elements can be for instance provided *in cis* (on the same plasmid) or *in trans* (on a separate plasmid). Viral vectors may require helper viruses or packaging lines for large-scale transfection. Vectors may contain further elements such as e.g. enhancer elements (e.g. viral, eukaryotic), introns, and viral origins of plasmid replication for replication in mammalian cells. According to the current invention, expression vectors typically have a promoter sequence that drives expression of the POI. Expression of the POI and/or selective marker protein may be constitutive or regulated (e.g. inducible by addition or removal of small molecule inductors). Preferred regulatory sequences for mammalian host cell expression include viral elements that direct high levels of expression of a POI in mammalian cells, such as regulatory elements, promoters and/or enhancers derived from cytomegalovirus (CMV), Simian Virus 40 (SV40), adenovirus, (e.g., the adenovirus major late promoter Ad LP) or polyoma. For further description of viral regulatory elements, and sequences thereof, see e.g., U.S. 5.168.062 by Stinski, U.S. 4,510,245 by Bell et al. and U.S. 4,968,615 by Schaffner et al..

**"Promoters"** are regulatory elements. Suitable promoters according to the current invention are all promoters which are compatible with the expression system and which lead to the intended expression levels. Some promoters are called constitutive as their activity is dependent on the availability of RNA polymerase only, while others are regulated, becoming active in the cell only in response to specific stimuli. The strength of a promoter can be determined as known in the art. Preferably, the strength of the promoter is assessed in the applied expression system with a reporter protein as read-out. A weak promoter yields low transcription levels and thus low levels of protein product. Strong promoters induce high levels of protein translation with respect to an expression system and include for instance viral promoters, such as a CMV promoter. Variables that may influence the strength of a promoter may comprise the affinity for RNA polymerase or the expression system.

A first promoter is called **"weaker"** than a second promoter, if within the same expression vector and expression system less reporter protein is obtained from a reporter gene, if this reporter gene is under the control of the first promoter than if it is under the control of the second promoter. For instance for the expression systems at hand, truncated SV40 promoter such as the Delta3SV40E promoter are weaker than the corresponding non-truncated Simian Virus 40 (SV40) promoter. The recombinant expression vectors can also include further selective marker genes (see e.g., U.S. 4,399,216, 4,634,665 and U.S. 5, 179,017). The further selective marker genes can be or can be based on genes that confer resistance to drugs such as G418, Puromycin, hygromycin, blasticidin, zeocin/bleomycin or methotrexate. For example, the dihydrofolate reductase (DHFR) gene confers resistance to methotrexate, neo gene confers resistance to G418, the bsd gene from Aspergillus terreus confers resistance to blasticidin, Puromycin N-acetyl-transferase confers resistance to Puromycin, the Sh ble gene product confers resitance to zeocin, and resistance to hygromycin is conferred by the E. coli hygromycin resistance gene (hyg or hph). Furthermore, the further selective marker genes can be or can be based on selective marker genes that exploit auxotrophies, such as Glutamine Synthetase (Bebbington et al., Biotechnology (N Y). 1992 Feb; 10(2):169-75) or P5CS.

The terms **"host cell", "host cell line"**, and **"host cell culture"** are used interchangeably and refer to a cell or cells into which exogenous nucleic acid molecule(s), polynucleotide(s) and/or (expression) vector(s) have been introduced, including the progeny of such cells. The term furthermore comprises **"transformed cells"**, **"transfected cells"**, and **"transduced cells"**, which include the primary transformed/transfected/transduced cell and progeny derived therefrom without regard to the number of passages. Progeny may or may not be completely identical in nucleic acid content to a parent cell, hence, they may contain mutations. Mutant progeny that have the same function or biological activity as screened or selected for in the originally transformed cell are included herein.

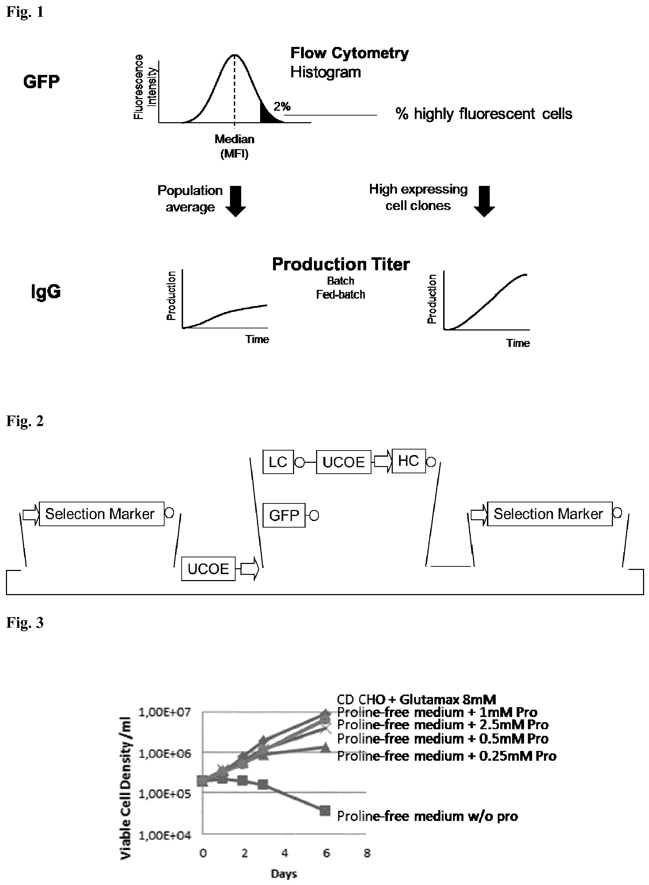

The term **"stringency"** refers to the selective pressure which is applied to cells. The more stringent a selection system is, the more cells with low expression of the selection marker protein die. The cells that survive the stringent procedure are characterized by higher expression levels of the positive selection marker. More stringent selection systems usually create fewer clones after transfection, whereas in the optimal case these clones display higher POI expression values due to a correlation between the expression levels of POI and selective marker. Different readouts can be used to characterize a selection system with respect to stringency (see also Fig. 1) and protein expression performance. These readouts are described in the following for IgG and green fluorescent protein (GFP) as exemplary POIs, but can be easily adapted for various user-defined POIs. Firstly, the kinetics of cell population recovery or cell viabilities at certain stages after adding selection pressure is an indirect way of measuring selection stringency conditions (e.g. the burden associated with a particular condition). Secondly, using flow cytometry, the Median Fluorescence Intensity (MFI) of the cell population can be determined, and is for example measured after recovery from transfection and subsequent stable selection. This is a measure for the average expression level of a fluorescent reporter POI such as GFP within that population. MFIs of a reference population within an experiment can be compared to those having undergone different selection test conditions. For POI(s) without inherent fluorescence, staining with a respective fluorescent antibody enables assessment of the MFI for a population of cells. Thirdly, the batch or fed-batch titer after transfection with human IgG1 or another POI and subsequent appropriate fermentation can be determined. This titer will reflect e.g. the average IgG expressing capability of the stably transfected cell population. Fourthly, flow cytometry of the cell population after cell population recovery can be exploited to determine the percentage of cells expressing a fluorescent reporter POI such as GFP in high amounts (% high expressers or highly fluorescent cells). As discussed before for POI(s) without inherent fluorescence, staining with the respective fluorescent antibody can be performed in the alternative. Using the same threshold within an experiment, these percentages can be compared between populations derived from different test conditions relative to a reference population. By using GFP as a test molecule, this allows to easily identify the impact of different conditions not only on average expression capability of the cell population as a whole but also to study impact on the most attractive high expressing subpopulation. The same way the MFI of the whole cell population is related to the titer of IgG achieved by the whole population in relative terms, the percentage of highly fluorescent cells is assumed to be related to the titers of the IgG achieved in high expressing clonal cells. In the case of IgG (or any recombinant POI to be produced in high amounts), this highly expressing population would typically be cloned to obtain highly producing clonal cell lines. The higher the frequency of highly expressing clones and the higher the level of protein expression in that high expressing clones, the more attractive is such a condition as a procedure to obtain highly productive cell lines for manufacturing.

### Embodiments

The current invention comprises tools and methods to allow for a high stringency P5CS based selection of proline-auxotrophic cells for efficient protein production. The method circumvents safety challenges known e.g. from antibiotic based systems, keeps the screening effort manageable and enables high production levels of a POI as demonstrated exemplary for IgG1 antibody and green fluorescent protein (GFP). Compared to conventional P5CS based selection systems, the stringency is increased, and also protein production is superior not only with regard to P5CS based selection systems but also relative to state of the art antibiotic selection systems.

Combining selection based on a) selective marker P5CS/essentially proline-free selection medium and b) DHP as a stringency agent can be seen as a two part selection system. In contrast to antibiotics based systems and other metabolic systems, wherein antibiotics or toxic small molecules might pose safety challenges, the full or a part of the stringency of the system may be uphold until harvesting the protein. As described in Example 9, the proline synthesized by a transfected cell upon expression of P5CS is enough to sustain a high level of production titers of a secreted protein of choice without slowing down the growth and proliferation of the cells due to increased metabolic burden.

According to the current invention, a host cell line with a natural or acquired, transient or persisting deficiency in the synthesis of proline is used for protein production. In the essential absence of exogenous proline, e.g. in an essentially proline-free cell culture medium, proline-auxotrophic cells lack proline and will eventually die, e.g. from the resulting deficiency in protein biosynthesis. For example, in an essentially proline-free medium, the vast majority of CHO cells die within ten days (Kao and Puck, 1967, see also Example 13 with Fig. 3 and Fig. 4).

However, using the metabolic selection system according to the current invention, the proline-auxotrophy can be rescued for some of the cells. For example, a polynucleotide encoding for a functional P5CS as a selective marker can be introduced into a proline-auxotrophic cell. Where a successful integration of at least the selective marker gene occurs, this may lead to the expression of the functional P5CS. If sufficient amounts of functional P5CS are synthesized, the enzyme enables the production of proline from metabolic precursors such as glutamate and its derivatives, thereby rescuing the proline auxotrophy of the cell. Upon exposure to selective growth conditions such as a cell culture medium without proline, only those cell clones will survive, wherein the proline-auxotrophy has been rescued.

A variety of compounds, including L-Proline, L-ornithine, L-Azetidine-2-carboxylic acid, trans-4-Hydroxy-L-Proline, cis-4-Hydroxy-L-Proline, Thiazolidine-2-carboxylic acid, was tested for their suitability to increase both, selection stringency and POI expression performance. While few compounds were found to increase stringency, none of these compounds could likewise increase the expression performance (see Example 2, Example 3 and Example 5).

Instead, for the selection system of the current invention, wherein the metabolic selectable marker is a functional P5CS, it was found that only the presence of DHP leads to a surprising increase not only in selection stringency but also in expression performance as demonstrated in Example 6. In particular, recovery of cell populations selected in the essential absence of proline and in the presence of DHP was slower compared to the control. In addition to an increase in selective pressure, as indicated also by a reduced percentage of cells surviving the selection procedure, presence of DHP leads to a surprising increase in protein titers for the POI. In brief, addition of 0.2 mM DHP resulted in a 37 % increase in MFI as compared to a Puromycin-selected population (Table 10). Similarly, the frequency of high fluorescing cells was increased by 71% (Table 11). This observation of a combined effect of DHP for the selection system at hand was particularly surprising, because Wasmuth and Caskey (Wasmuth and Caskey, Life Sci. 1978 May 1;22(17):1469-80) describe no effect of DHP on the synthesis of proline from glutamic acid. Instead, DHP has been shown to be a substrate for a different enzyme, P5C reductase, an inhibitor of prolyl hydroxylase and an inhibitor of Proline Oxidase. The combined effect of an increase in selection stringency and an increase in protein titers elicits a decrease in screening effort, because only cells with favorable production profiles survive, and therefore fewer cells have to be screened in order to find the clones with highest production.

According to a first aspect, the current invention comprises the use of 3,4-dehydroproline (DHP) and/or at least one of its salts in a metabolic selection system, wherein the metabolic selective marker is a functional pyrroline-5-carboxylate synthase (P5CS).

A metabolic selection system according to the first aspect is a system for selection of a subpopulation of cells from a pool of cells, wherein the subpopulation of cells can be distinguished by means of a metabolic selective marker.

Suitable cells for such a metabolic selection system according to the first aspect are cells with a natural or acquired (e.g. by genetical engineering), permanent or temporary proline auxotrophy. In some preferred embodiments the cells of the metabolic selection system according to the first aspect are eukaryotic. Protein expression in eukaryotic cells facilitates appropriate post-translational modification of the produced proteins. In some preferred embodiments, the cells of the metabolic selection system according to the first aspect are cells that can be grown in suspension culture to facilitate protein production. In a preferred embodiment the cells of the metabolic selection system according to the first aspect are eukaryotic and can be grown in suspension culture.

In some embodiments, the cells of the metabolic selection system according to the first aspect are selected from the list comprising NS0 myeloma cells, COS cells, HEK293 cells, HEK293T cells, HEK293-EBNA cells, HEK293E cells, HEK293-6E cells, HEK293-Freestyle cells, HKB1 1 cells, BHK21 cells, Expi293F cells, 293EBNALT75 cells, CAP cells, CAP-T cells, EB66 cells, SP2 cells, and cells from another mammalian cell line, and have been engineered to obtain a suitable proline auxotrophic mammalian host cell line. Suitable methods to engineer the cells to introduce a proline auxotrophy comprise methods for knockout of P5CS and are well known in the art.

In some preferred embodiments, the cell line used for the metabolic selection system according to the first aspect is characterized by a natural proline auxotrophy. In some preferred embodiments, the cells of the metabolic selection system according to the first aspect are Chinese Hamster Ovary (CHO) cells, such as CHO-K1, CHO-S, CHO-K1SV, CHO Freestyle, CHOEBNALT85, CHOS-XE, CHO-DG44, CHO-DXB11 or CHO-3E7 cells. CHO cell lines are commonly used for eukaryotic protein expression due to their favorable properties e.g., regarding protein folding, protein yields and culturing properties.

In some preferred embodiments, the cells are selected after culturing them in a cell culture medium, which is essentially proline-free with respect to the cultured cells. Essential absence of proline in the environment or culture medium of the selection system is important, as otherwise all cells are able to survive, hence positive selection would not be possible with a metabolic selection system based on P5CS. In a preferred embodiment according to the first aspect, the cells are selected after culturing them in a cell culture medium for selection, wherein the cell culture medium for selection is prepared without adding proline.

In some preferred embodiments according to the first aspect, the functional P5CS is P5CS. In a preferred embodiment according to the first aspect, the functional P5CS is derived from human, mouse, Chinese hamster or another mammal. In some embodiments according to the first aspect, the functional P5CS is a long isoform of murine P5CS (UniProtKB accession number: Q9Z110-1). In some preferred embodiments according to the first aspect, the functional P5CS is a short isoform of murine P5CS (UniProtKB accession number: Q9Z110-2). In some preferred embodiments according to the first aspect, the functional P5CS is P5CS human long isoform P54886-1. In some embodiments according to the first aspect, the functional P5CS is P5CS human short isoform P54886-2. In some embodiments according to the first aspect, the functional P5CS is P5CS Chinese Hamster isoform G3HXX8-1 or A0A061IBJ4-1.

In some embodiments according to the first aspect, the selective marker is encoded by a corresponding selective marker gene. In some embodiments according to the first aspect, the selective marker gene is or forms part of a polynucleotide which is introduced into at least one host cell, leading to transient or stable expression of the corresponding selective marker protein.

The stringency of the selection system according to the current invention can be increased or further increased by reducing the amount and/or activity of the functional selective marker. One option to reduce the amount of functional selective marker is by using one or more specific control elements of protein expression, such as specific promoters as discussed in more detail for the second and third aspect of the current invention. One option to reduce the amount of functional selective marker is by using a selective marker with a reduced activity. One option to reduce the activity of a functional selective marker is by changing the protein sequence, e.g. by specific mutation or any another technique known in the art. In some embodiments according to the first aspect, the functional selective marker is a mutated P5CS, where the activity is lower than the activity of short isoform of mouse P5CS. The activities of the selective marker can be easily compared as known in the art, e.g. by mass spectrometric determination of the amount of generated product or intermediates, such as glutamate 5-semialdehyde, pyrroline-5-carboxylate, or proline.

In a preferred embodiment according to the first aspect, use of DHP and/or at least one of its salts comprises contacting the cells of the selection system with DHP and/or at least one of its salts. Exposing the cells of the selection system to DHP results in an increase in stringency and has a beneficial impact on the protein expression performance of the selection system. In a preferred embodiment according to the first aspect, use of DHP and/or at least one of its salts comprises selection of cells after culturing them in a cell culture medium for selection, where the cell culture medium for selection at least temporary comprises DHP and/or at least one of its salts.

In some preferred embodiments according to the first aspect, the stringency of the selection system is increased or further increased by increasing or modulating the concentration of the DHP and/or at least one of its salts. In a preferred embodiment according to the first aspect, the concentration or the concentration gradient of the DHP and/or at least one of its salts is optimized for the respective selection system. Without being bound by theory, the optimal concentration or concentration gradient for DHP and/or at least one of its salts depends on the realization of the selection system and the expression and type of the selective marker. Positioning of the expression cassette and regulatory elements may likewise have an impact on the optimal concentration or concentration gradient of the DHP and/or at least one of its salts (see Example 11). A procedure for optimization of the concentration of DHP and/or at least one of its salts is given in Example 7, and can be easily adapted by a person skilled in the art for different polynucleotides or vectors, cell lines or otherwise changed parameters of the selection system.

In some embodiments according to the first aspect, concentration of DHP and/or at least one of its salts varies over time. In some embodiments, concentration of DHP and/or at least one of its salts decreases during the selection process. In some embodiments, concentration of DHP and/or at least one of its salts increases during the selection process. In one embodiment, DHP and/or at least one of its salts is present throughout the whole selection process until harvesting the POI(s).

In some embodiments, the concentration of DHP and/or at least one of its salts is at least temporary between 0.001 mM and 100 mM. In some embodiments, the concentration of DHP and/or at least one of its salts is at least temporary between 0.01 mM and 10 mM. In some preferred embodiments, the concentration of DHP and/or at least one of its salts is at least temporary between 0.1 mM and 1 mM. In some preferred embodiments, the concentration of DHP and/or at least one of its salts is at least temporary about n mM, and n is selected from the list consisting of 0.001, 0.0011, 0.0012, 0.0013, 0.0014, 0.0015, 0.0016, 0.0017, 0.0018, 0.0019, 0.002, 0.0021, 0.0022, 0.0023, 0.0024, 0.0025, 0.0026, 0.0027, 0.0028, 0.0029, 0.003, 0.0031, 0.0032, 0.0033, 0.0034, 0.0035, 0.0036, 0.0037, 0.0038, 0.0039, 0.004, 0.0041, 0.0042, 0.0043, 0.0044, 0.0045, 0.0046, 0.0047, 0.0048, 0.0049, 0.005, 0.0051, 0.0052, 0.0053, 0.0054, 0.0055, 0.0056, 0.0057, 0.0058, 0.0059, 0.006, 0.0061, 0.0062, 0.0063, 0.0064, 0.0065, 0.0066, 0.0067, 0.0068, 0.0069, 0.007, 0.0071, 0.0072, 0.0073, 0.0074, 0.0075, 0.0076, 0.0077, 0.0078, 0.0079, 0.008, 0.0081, 0.0082, 0.0083, 0.0084, 0.0085, 0.0086, 0.0087, 0.0088, 0.0089, 0.009, 0.0091, 0.0092, 0.0093, 0.0094, 0.0095, 0.0096, 0.0097, 0.0098, 0.0099, 0.01, 0.011, 0.012, 0.013, 0.014, 0.015, 0.016, 0.017, 0.018, 0.019, 0.02, 0.021, 0.022, 0.023, 0.024, 0.025, 0.026, 0.027, 0.028, 0.029, 0.03, 0.031, 0.032, 0.033, 0.034, 0.035, 0.036, 0.037, 0.038, 0.039, 0.04, 0.041, 0.042, 0.043, 0.044, 0.045, 0.046, 0.047, 0.048, 0.049, 0.05, 0.051, 0.052, 0.053, 0.054, 0.055, 0.056, 0.057, 0.058, 0.059, 0.06, 0.061, 0.062, 0.063, 0.064, 0.065, 0.066, 0.067, 0.068, 0.069, 0.07, 0.071, 0.072, 0.073, 0.074, 0.075, 0.076, 0.077, 0.078, 0.079, 0.08, 0.081, 0.082, 0.083, 0.084, 0.085, 0.086, 0.087, 0.088, 0.089, 0.09, 0.091, 0.092, 0.093, 0.094, 0.095, 0.096, 0.097, 0.098, 0.099, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, and 1. In this particular context, the term "about" refers to +/- 20 %. In some preferred embodiments according to the first aspect, the concentration of DHP and/or at least one of its salts in the medium is about n mM, and n is selected from the list consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100. In this particular context, the term "about" refers to +/- 20 %.

According to a second aspect, the current invention comprises a method for culturing and screening eukaryotic cells for the expression of high levels of one or more POI, the method comprising (i) introducing one or more polynucleotide(s) into a eukaryotic cell, wherein the one or more polynucleotide(s) encode(s) a functional P5CS and at least one POI, (ii) culturing the cell in an essentially proline-free cell culture medium, and (iii) culturing the cell in the presence of DHP and/or at least one of its salts.

Suitable eukaryotic cells to be employed in the method according to the second aspect include cells with a natural or acquired (e.g. by genetical engineering) permanent or temporary proline auxotrophy. In some embodiments, the eukaryotic cells according to the second aspect are selected from the list comprising NS0 myeloma cells, COS cells, HEK293 cells, HEK293T cells, HEK293-EBNA cells, HEK293E cells, HEK293-6E cells, HEK293-Freestyle cells, HKB1 1 cells, BHK21 cells, Expi293F cells, 293EBNALT75 cells, CAP cells, CAP-T cells, EB66 cells, SP2 cells, and cells from another mammalian cell line, and have been engineered to obtain a suitable proline auxotrophic mammalian host cell line. Suitable methods to engineer the cells to introduce a proline auxotrophy include methods for knockout of P5CS and are well known in the art (see for instance Kühn and Wurst (2009) Gene Knockout Protocols, Methods in Molecular Biology).

Employing eukaryotic cells with a natural proline auxotrophy may be advantageous, because no further engineering is necessary. In some preferred embodiments, the eukaryotic cells according to the second aspect are Chinese Hamster Ovary (CHO) cells, such as CHO-K1, CHO-S, CHO-K1SV, CHO Freestyle, CHOEBNALT85, CHOS-XE, CHO-DG44, CHO-DXB11 or CHO-3E7 cells. CHO cell lines are commonly used for eukaryotic protein expression due to their favorable properties e.g., regarding protein folding, protein yields and culturing properties.

Introducing one or more polynucleotide(s) into a eukaryotic cell can occur and is specifically suggested to occur by any method that is compatible with the respective format, e.g. with the type of cell and polynucleotide(s). In a preferred embodiment according to the second aspect, introducing the polynucleotide into the eukaryotic cell according to the second aspect is carried out by transfection or viral transduction. In some preferred embodiments, introducing the polynucleotide according to the second aspect is carried out by electroporation, nucleofection, calcium-phosphate precipitation, lipofection, polycation-based transfection such as polyethlylenimine (PEI)-based transfection or DEAE-dextran transfection. In a preferred embodiment, introducing the polypeptide into the eukaryotic cell occurs by nucleofection.

In some preferred embodiments, the one or more polynucleotide(s) according to the second aspect is or forms part of an expression vector. In a particular embodiment, multiple polynucleotides forming part of one or more expression vectors are introduced into the same cell. In some embodiments, multiple vectors are introduced into the same cell. In some preferred embodiments, the polynucleotide according to the method of the second aspect is a polynucleotide according to the third aspect.

In a preferred embodiment according to the second aspect, the encoded functional P5CS is P5CS. In a preferred embodiment according to the second aspect, the functional P5CS is derived from human, mouse, Chinese hamster, or another mammal. In some preferred embodiments according to the second aspect, the functional P5CS is murine long isoform Q9Z110-1. In some preferred embodiments according to the second aspect, the functional P5CS is murine short isoform Q9Z110-2. In a preferred embodiment according to the second aspect, the functional P5CS is human long isoform P54886-1 or human short isoform P54886-2. In some embodiments according to the second aspect, the functional P5CS is P5CS Chinese Hamster isoform G3HXX8-1 or A0A061IBJ4-1. In some preferred embodiments according to the second aspect, the functional P5CS has been engineered or mutated.

The at least one POI encoded by the polynucleotide according to the second aspect can be any modified or unmodified linear chain of amino acids. In a particular embodiment the at least one POI encoded by a polynucleotide according to the second aspect is or comprise(s) a peptide, a protein or a fusion protein. In some embodiments at least one POI encoded by a polynucleotide according to the second aspect is or comprise(s) an antibody or an antibody fragment, for example as described in Example 4 or as specified in the definitions section. In some embodiments, at least one of the at least one POI comprises a cleavage site. In some embodiments, at least one of the at least one POI is modified during or after expression.

In some preferred embodiments, the method according to the second aspect further comprises the optional step of isolating, recovering or harvesting the at least one POI. In some preferred embodiments, the at least one POI is a product of the method according to the second aspect and can be subsequently isolated from a selected cell by any protein extraction technique known in the art (see for instance Cutler (Ed.), Protein Purification Protocols, Methods in Molecular Biology 244, Humana Press, 2004). In some preferred embodiments, a cell selected by means of the method according to the second aspect is a product of said method and can be subsequently propagated in cell culture as known in the art, to provide a replenishing resource for POI production.

In some embodiments, the at least one POI encoded by a polynucleotide according to the second aspect is or comprise(s) a secreted protein (see also Example 4), an intracellular protein, an extracellular protein, a surface protein, a nuclear protein, a membrane protein, a transcription factor, a growth factor, a hormone, a cytokine, a cell adhesion protein, or a cell signaling protein. In some embodiments the at least one POI according to the second aspect can be used for the treatment of a disease. In some embodiments, the at least one POI is or comprise(s) a read-out protein such as a fluorescent marker protein to evaluate the performance of the system.

In some preferred embodiments, the polynucleotide introduced according to the method of the second aspect is a polynucleotide comprising a nucleic acid sequence encoding a functional P5CS and at least one POI, wherein the expression of the functional P5CS and the expression of at least one POI are under the control of different promoters, and wherein the promoter controlling said functional P5CS is weaker than the promoter controlling said at least one POI.

To ensure the predictive power of the selection marker with respect to protein production, the expression of the selection marker must not be independent from the expression of POI. One option to ensure this correlation according to the current invention is by using a polynucleotide encoding for both, selective marker and POI. It is however not necessary that selective marker and POI expression are under the control of the same regulatory elements. Instead, if different promoters are used, as described for this invention, this leads to a surprising increase in the overall performance of the selection system.

In some preferred embodiments, the polynucleotide according to the method of the second aspect comprises a nucleic acid sequence encoding a functional P5CS and at least one POI, wherein the expression of the functional P5CS and the expression of at least one POI are under the control of different promoters, and wherein the promoter controlling the expression of the functional P5CS is a weak promoter, such as a SV40 promoter, a truncated SV40 promoter or a Delta3SV40E promoter.

In some embodiments the polynucleotide of the method according to the second aspect encodes for a single POI. In some embodiments, the polynucleotide of the method according to the second aspect encodes for two, three, four, five or more POI. In some embodiments, the polynucleotide of the method according to the second aspect encodes for two, three, four, five or more POI and at least two of the POI are different from each other. In a preferred embodiment the resulting expression of the one or more POI(s) is either transient or stable.

The method according to the second aspect further comprises culturing the cell line in an essentially proline-free cell culture medium for said cell line. In a preferred embodiment, the essentially proline-free cell culture medium according to the second aspect was prepared without addition of proline. In some embodiments, the essentially proline-free cell culture medium according to the second aspect was prepared without addition of proline, but the cultured cells have secreted proline into the medium after contacting the medium with the cells.

Culturing of eukaryotic cells is well known in the art (Helgason and Miller (Eds.), 2013, Basic Cell Culture Protocols, Methods in Molecular Biology 946, Humana Press). Standard conditions comprise for instance growing the cells under sterile conditions at 37°C and 5% CO2. A suitable medium according to the second aspect can be or can be based on any medium for cell culture which is compatible with the specific needs of the used eukaryotic cells. In particular, the medium should support a robust cell growth in batch culture to allow for high titers of POI expression.

In a preferred embodiment, according to the second aspect, the medium is fully chemically defined. Chemically defined media allow for reproducible automation of biotechnological protein production processes. Compatible media for cell culture are well described for the respective cell lines and can be adapted for the specific needs by the skilled person. Typically, adapting these media comprises omitting proline when combining the ingredients of the medium. Furthermore, customized media can be easily established by the person skilled in the art. In some preferred embodiments according to the second aspect, wherein the eukaryotic cells according to the second aspect are CHO cells, the essentially proline-free cell culture medium is based on a medium for culturing CHO cells, such as EX-CELL CD CHO Fusion medium. Further compounds or components can be added to the cell culture medium which forms the base of the essentially proline-free cell culture medium. For example, antibiotics can be added to the medium, in particular where the selection system employs an additional selection marker which conveys antibiotic resistance.

In some embodiments, the essentially proline-free cell culture medium is essentially free of ornithine. A cell culture medium is essentially free of ornithine, if the cell culture medium was prepared without adding ornithine. As described in Example 3, cell populations selected with proline-free medium containing additional ornithine displayed a significantly decreased expression performance. In some preferred embodiment the essentially proline-free cell culture medium is based on EX-CELL CD CHO Fusion medium and is essentially free of ornithine.

In some preferred embodiments, the cell culture medium according to the second aspect comprises GlutaMAX or glutamine. In some preferred embodiments according to the second aspect, wherein the eukaryotic cells according to the second aspect are CHO cells, the essentially proline-free selection medium is based on EX-CELL CD CHO Fusion medium, and/or comprises 1 - 50 mM GlutaMAX or glutamine, preferably about 1, 2, 3, 4, 5, 6, 7, 8, 9, 10, 11, 12, 13, 14, 15, 16, 17, 18, 19 or 20 mM GlutaMAX and/or glutamine, and/or is essentially free of ornithine. In this particular context the term about refers to +/- 20 %.

In some embodiments, the conditions of culturing such as suspension culture facilitate up-scaling and large scale production. In a preferred embodiment, the eukaryotic cells according to the second aspect are cells that can be grown in suspension culture to facilitate protein production and/or the medium according to the second aspect supports growth in suspension culture.

In some preferred embodiments, the essentially proline-free cell culture medium employed according to the second aspect is an essentially proline-free cell culture medium according to the fifth aspect. In some embodiments, the essentially proline-free cell culture medium at least temporary comprises DHP and/or at least one of its salts. In a preferred embodiment according to the second aspect, the culturing the cells in the presence of DHP and/or at least one of its salts occurs at a concentration or concentration gradient of the DHP and/or at least one of its salts which has been optimized for the specific conditions of the method.

As discussed previously for the first aspect, the optimal concentration and/or concentration gradient is a function of the specific steps and embodiments of the method. A procedure for optimization of the concentration of DHP and/or at least one of its salts is given in Example 7, and can be easily adapted by a person skilled in the art for different polynucleotides or vectors, cell lines or otherwise changed parameters. In some embodiments according to the second aspect, concentration of DHP and/or at least one of its salts varies over time. In some embodiments, concentration of DHP and/or at least one of its salts decreases over time. In some embodiments, concentration of DHP and/or at least one of its salts increases over time. In a preferred embodiment, DHP and/or at least one of its salts is present until the POI(s) is/are harvested. In some embodiments, the concentration of DHP and/or at least one of its salts is at least temporary between 0.001 mM and 100 mM. In some embodiments, the concentration of DHP and/or at least one of its salts is at least temporary between 0.01 mM and 10 mM. In some preferred embodiments, the concentration of DHP and/or at least one of its salts is at least temporary between 0.1 mM and 1 mM. In some preferred embodiments, the concentration of DHP and/or at least one of its salts is at least temporary about n mM, and n is selected from the list consisting of 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, and 1. In this particular context, the term "about" refers to +/- 20 %. In some preferred embodiments, the concentration of DHP and/or at least one of its salts according to the second aspect is at least temporary about n mM, and n is selected from one of the lists disclosed for the concentrations for DHP and/or its salts according to the first aspect. In some preferred embodiments according to the second aspect, the concentration of DHP and/or at least one of its salts in the medium is about n mM, and n is selected from the list consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100. In this particular context, the term "about" refers to +/- 20 %.

According to a third aspect of the current invention, there is provided a polynucleotide comprising a nucleic acid sequence encoding a functional P5CS and at least one POI, wherein the expression of the functional P5CS and the expression of at least one POI are under the control of different promoters, and wherein the promoter controlling said functional P5CS is weaker than the promoter controlling said at least one POI, and/or wherein the promoter controlling the expression of the functional P5CS is a weak promoter, such as a SV40 promoter, a truncated SV40 promoter or a Delta3SV40E promoter.

According to the current invention, another way to render the P5CS selection system superior regarding selective pressure and protein yields compared to antibiotic based selection systems is by controlling the amount of produced selective marker by means of an individual promoter. If the P5CS expression cassette is under the control of a weak promoter such as a SV40 promoter, a weakened SV40 promoter, such as truncated Delta3SV40E promoter, there is an increase in selective power of the selection system and if the at least one POI is under the control of a stronger promoter, there is also a surprising increase in protein expression performance as demonstrated in Example 12.

Remarkably, the use of a weakened promoter, such as a weakened SV40 promoter, thus already led to a superior expression performance for the POI, even in the absence of DHP, as compared to an antibiotics based selection. At the same time, presence of optimized levels of DHP had a further beneficial impact on stringency and expression performance for the POI. Without being bound by theory, these observations were consistent with a truncated, weakened promoter yielding lower amounts of P5CS. Without being bound by theory, the amount and activity of selective marker P5CS seems to impact the optimal concentration window of DHP for defining the selective growth conditions.

In a preferred embodiment, the polynucleotide according to the third aspect is a eukaryotic expression vector. Of note, it is an inherent feature of the vector or polynucleotide, that different functional P5CS selection markers can be combined with various POI(s), various promoters and various further elements in a modular fashion. In consequence, each embodiment describing the selection marker can be and is explicitly suggested to be combined with each embodiment describing the POI and/or with each embodiment describing the promoter(s) or regulatory elements, and/or with each embodiment describing the vector backbone elements, except if the skilled person would consider the embodiments to be obviously incompatible.

In some embodiments, the vector or polynucleotide comprises at least one constitutive promoter. In some embodiments, the vector or polynucleotide comprises at least one regulated promoter. In some embodiments according to the third aspect, the vector or polynucleotide comprises at least one weak promoter, such as a SV40 promoter, a truncated SV40 promoter or a Delta3SV40E promoter. In some embodiments according to the third aspect, the expression of the at least one functional P5CS is under the control of a weak promoter, such as a SV40 promoter, a truncated SV40 promoter or a Delta3SV40E promoter.

In a preferred embodiment, the polynucleotide according to the third aspect comprises a nucleic acid sequence encoding a functional P5CS and at least one POI, wherein the expression of the functional P5CS and the expression of at least one POI are under the control of different promoters, and wherein the promoter controlling the expression of the functional P5CS is a weak promoter, such as a SV40 promoter, a truncated SV40 promoter or a Delts3SV40E promoter.

In some embodiments according to the third aspect, the vector or polynucleotide comprises at least one strong promoter, such as a wildtype CMV promoter, e.g. a guinea pig CMV regulatory element (promoter A). In some preferred embodiments according to the third aspect, the expression of at least one of the at least one POI is under the control of a strong promoter. In some of these embodiments, a CMV promoter (e.g. from mouse, rat, human or guinea pig CMV) controls expression of at least one of the at least one POI.

In some preferred embodiments according to the third aspect, the promoter controlling expression of the functional P5CS is weaker than promoter A. In some embodiments according to the third aspect, the vector or polynucleotide comprises a Delta3SV40E promoter controlling expression of the functional P5CS, and at least a second promoter controlling expression of at least one POI.

In a preferred embodiment, the polynucleotide according to the third aspect comprises a nucleic acid sequence encoding a functional P5CS and at least one POI, wherein the expression of the functional P5CS and the expression of at least one POI are under the control of different promoters, and wherein the promoter controlling said functional P5CS is weaker than the promoter controlling said at least one POI. In some embodiments according to the third aspect, the vector or polynucleotide comprises a Delta3SV40E promoter controlling expression of the functional P5CS, a second promoter controlling expression of a first POI and a third promoter controlling expression of a second POI.

In some preferred embodiments according to the third aspect, the encoded functional P5CS is P5CS. In some preferred embodiments according to the third aspect, the encoded functional P5CS is derived from human, mouse, Chinese hamster, or another mammal. In some preferred embodiments according to the third aspect, the encoded functional P5CS is murine long isoform Q9Z110-1. In some preferred embodiments according to the third aspect, the encoded functional P5CS is murine short isoform Q9Z110-2. In some preferred embodiments according to the third aspect, the encoded functional P5CS is human long isoform P54886-1 or human short isoform P54886-2. In some preferred embodiments according to the third aspect, the encoded functional P5CS has been engineered or mutated. In some preferred embodiments according to the third aspect, the vector or polynucleotide comprises a nucleic acid sequence as set forth in SEQ ID NO:2 and/or SEQ ID NO:5.

In some preferred embodiments according to the third aspect, the promoter controlling the P5CS is Delta3SV40E promoter and the encoded functional P5CS is murine long isoform Q9Z110-1 or murine short isoform Q9Z110-2. In a preferred embodiment according to the third aspect, the vector or polynucleotide comprises a nucleic acid sequence as set forth in SEQ ID NO:3 and/or SEQ ID NO:6.

In a preferred embodiment according to the third aspect, the polynucleotide comprises a nucleic acid sequence as set forth in SEQ ID NO:2, SEQ ID NO:3, SEQ ID NO:5 and/or SEQ ID NO:6.

In some embodiments according to the third aspect, the polynucleotide further encodes for an additional selective marker protein, such as a further metabolic selective marker or an antibiotic selective marker.

Nucleic acid sequences encoding for such an antibiotic selective marker can be for example, dihydrofolate reductase (DHFR) gene confering resistance to methotrexate, neo gene confering resistance to G418, the bsd gene from Aspergillus terreus confering resistance to blasticidin, Puromycin N-acetyl-transferase conferring resistance to Puromycin, the Sh ble gene confering resitance to zeocin, and the E. coli hygromycin resistance gene (hyg or hph) conferring resistance to hygromycin.

In some embodiments, the vector or polynucleotide according to the third aspect encodes for one POI. In some embodiments, the vector or polynucleotide according to the third aspect encodes for two, three, four, five or more POIs. In some of these embodiments, the two or more POI are different from each other.

In a particular embodiment according to the third aspect, the encoded at least one POI is or comprises a peptide, a protein, a fusion protein, an antibody, or a fragment thereof. In a particular embodiment according to the third aspect, the encoded at least one POI is or comprises a mature heavy chain and/or a mature light chain of an antibody.

In some embodiments according to the third aspect, the encoded at least one POI contains a protease cleavage site. In some embodiments according to the third aspect, the encoded at least one POI can be modified during or after expression. In some embodiments according to the third aspect, the encoded at least one POI is or comprises a secreted protein, an intracellular protein, an extracellular protein, a surface protein, a nuclear protein, a membrane protein, a transcription factor, a growth factor, a hormone, a cytokine, a cell adhesion protein, or a cell signaling protein.

In some preferred embodiments according to the third aspect, the encoded at least one POI can be used for the treatment of a disease. In some embodiments according to the third aspect, the encoded at least one POI is or comprises a read-out protein such as a fluorescent marker protein to evaluate the performance of the system.

In some embodiments according to the third aspect, the vector or polynucleotide further encodes for a reporter protein, such as a fluorescent protein. Examples for suitable reporter proteins include GFP or luciferase.

In some preferred embodiments according to the third aspect, the vector or polynucleotide further comprises one, two or more ubiquitous chromatin opening element(s) (UCOE). UCOEs can improve gene expression in stably transfected mammalian cells through effects on chromatin structure, preventing transgene silencing and allowing for gene expression irrespective of the chromosomal integration site.

In some preferred embodiments according to the third aspect, the vector or polynucleotide is a single or dual UCOE gpCMV-based vector, such as CET 1019 AS-hygro-SceI and CET 1019 AS-puro-SceI. CET 1019 AS-hygro-SceI and CET 1019 AS-puro-SceI are mammalian expression plasmid vectors containing the murine Rps3 UCOE upstream of a guinea pig CMV (gpCMV) promoter-enhancer element. In some preferred embodiments according to the third aspect, the vector or polynucleotide is a single or a dual UCOE hCMV-based vector, such as CET 1019 HS-hygro-SceI and CET 1019 HS-puro-SceI. CET 1019 HS-hygro-SceI and CET 1019 HS-puro-SceI are mammalian expression plasmid vectors containing the murine Rps3 UCOE upstream of a strong human cytomegalovirus (hCMV) immediate early promoter-enhancer element.

In some embodiments the vector or polynucleotide according to the third aspect is derived from one of the vector backbones listed in Table 1. In some preferred embodiments the vector or polynucleotide according to the third aspect is derived from one of the vector backbones listed in Table 1, wherein the sequence encoding GFP or human IgG1 has been at least partially replaced by a sequence encoding for the at least one POI.

In some preferred embodiments according to the third aspect, the vector or polynucleotide comprises a) a Delta3SV40E promoter controlling expression of a functional P5CS, b) a first UCOE element, and c) a second promoter, such as gpCMV promoter, controlling expression of a POI. In some preferred embodiments according to the third aspect, the vector or polynucleotide comprises a) a Delta3SV40E promoter controlling expression of a functional P5CS, b) a first UCOE element, c) a second promoter, such as promoter A, controlling expression of a first POI, d) a second UCOE element, and e) a third promoter, such as gpCMV promoter, controlling expression of a second POI.

In some preferred embodiments according to the third aspect, the vector or polynucleotide comprises a) a Delta3SV40E promoter controlling expression of the functional P5CS, such as murine short or long isoform of P5CS, b) a first UCOE element, c) a second promoter, such as gpCMV promoter, wherein the promoter controls expression of a first antibody or fragment thereof, such as mature light chain of an antibody, d) a second UCOE element, e) a third promoter, such as gpCMV promoter, wherein the promoter controls expression of a second antibody or fragment thereof, such as mature heavy chain of an antibody.

According to a fourth aspect, the current invention comprises a method for culturing and screening eukaryotic cells for the expression of high levels of at least one POI, the method comprising a) introducing a polynucleotide according to the third aspect of the invention into a eukaryotic cell, and b) culturing the cell in an essentially proline-free cell culture medium.

For the method for culturing and screening eukaryotic cells for the expression of high levels of at least one POI, the same requirements apply as described for the method according to the second aspect, except that presence of DHP or a salt thereof is optional. In consequence, each embodiment described for the second aspect, is also an embodiment of the method according to the fourth aspect, with the further restriction that a vector or polynucleotide according to the 3^{rd} aspect is used.

As discussed in Example 12, it was surprisingly found, that the use of a weak promoter controlling expression of functional P5CS led to a superior expression performance, even in the absence of DHP and/or its salt.

In a preferred embodiment, the method according to the 4^{th} aspect further comprises culturing the cells in the presence of DHP and/or at least one of its salts. As discussed for the third aspect, presence of optimized levels of DHP had a further beneficial impact on stringency and expression performance for the POI. The embodiments described for the second aspect which relate to the presence of optimized levels of DHP are also embodiments of the fourth aspect.

According to a fifth aspect, the current invention comprises an essentially proline-free cell culture medium comprising DHP and/or at least one of its salts for culturing and screening eukaryotic cells. The essentially proline-free medium according to the fifth aspect can be based on any medium for cell culture which is compatible with the specific needs of the cells it is employed for. In particular the medium according to the fifth aspect can be used in a method according to the second aspect to allow for stringent selection of cells in a high stringency metabolic selection and expression system.

Typically a medium according to the fifth aspect can be prepared by combining the ingredients of a base cell culture medium while omitting proline, and by adding DHP and/or at least one of its salts. In addition, further compounds or components can be added to the thereby prepared cell culture medium. For example, (further) antibiotics and/or antimycotic agents, such as penicillin-streptomycin can be added to the medium. Other compounds that can be added to the prepared cell culture medium are for example amino acids such as glutamine, vitamins, salts or balanced salt solution, proteins such as growth factors, hormones such as cholesterol, buffers, nucleosides such as thymidine, sodium hypoxanthine, aminopterin, antibiotics, antimycotics, trace elements, stem-cell supplements, 2-mercaptoethanol, lipids, fatty acids such as arachidonic acid, surfactants such as Pluronic F68, sugars such as mannose, glucose or sucrose, serum, pH indicator(s), or any other compound or compound mixture known in the art to be suitable for cell culture medium preparation.

In some preferred embodiments, the essentially proline-free cell culture medium according to the fifth aspect is based on a medium for culturing eukaryotic cells. In some embodiments, the essentially proline-free cell culture medium according to the fifth aspect is based on DMEM, RPMI 1640, MEM, IMDM, DMEM, DMEM/F-12, Opti-MEM or a serum-free cell culture medium. In particular, the medium should support a robust cell growth in batch culture to allow for high titers of protein expression. In some embodiments, the cell culture medium according to the fifth aspect is based on a medium which can be used for suspension culture of eukaryotic cells. In some embodiments, the cell culture medium or its base medium is suitable for culturing at least one of NS0 myeloma cells, COS cells, HEK293 cells, HEK293T cells, HEK293-EBNA cells, HEK293E cells, HEK293-6E cells, HEK293-Freestyle cells, HKB1 1 cells, BHK21 cells, Expi293F cells, 293EBNALT75 cells, CAP cells, CAP-T cells, EB66 cells, SP2 cells, and cells from another mammalian cell line. In some preferred embodiments, the base cell culture medium of the cell culture medium according to the fifth aspect is suitable for culturing at least one of Chinese Hamster Ovary (CHO) cells, such as CHO-K1, CHO-S, CHO-K1SV, CHO Freestyle, CHOEBNALT85, CHOS-XE, CHO-DG44, CHO-DXB11 or CHO-3E7 cells. In some preferred embodiments, the essentially proline-free cell culture medium is based on a medium for culturing CHO cells, such as EX-CELL CD CHO fusion medium.

Apart from adding specific compounds to a known cell culture medium, one or more compounds can likewise be omitted from the base formulation. In some embodiments according to the fifth aspect, the essentially proline-free cell culture medium is essentially free of ornithine. A cell culture medium is essentially free of a compound such as ornithine, if the cell culture medium was prepared without adding that compound. As described in Example 3, cell populations selected with proline-free medium containing additional ornithine displayed a significantly decreased expression performance.

In some preferred embodiments, the medium according to the fifth aspect is fully chemically defined. In some preferred embodiment the essentially proline-free cell culture medium is based on EX-CELL CD CHO Fusion medium and is essentially free of ornithine. In some embodiments, the cell culture medium according to the fifth aspect comprises GlutaMAX and/or L-glutamine. In some preferred embodiments according to the fifth aspect, the selection medium is based on EX-CELL CD CHO Fusion medium, and/or comprises GlutaMAX and/or L-glutamine, preferably 0.5 - 50 mM GlutaMax or glutamine, even more preferably 0.5, 1, 2, 3, 4, 5, 6, 7, 8, 9, or 10 mM GlutaMAX, and/or is essentially free of ornithine.

In some embodiments according to the fifth aspect, the concentration of DHP and/or at least one of its salts in the medium is > 0.0001 mM. In some preferred embodiments according to the fifth aspect, the concentration of DHP and/or at least one of its salts in the medium is between 0.001 mM and 100 mM. In some preferred embodiments according to the fifth aspect, the concentration of DHP and/or at least one of its salts in the medium is between 0.01 mM and 10 mM. In some embodiments according to the fifth aspect, the concentration of DHP and/or at least one of its salts in the medium is between 0.1 mM and 1 mM. In some preferred embodiments according to the fifth aspect, the concentration of DHP and/or at least one of its salts in the medium is about n mM, and n is selected from the list consisting of 0.001, 0.0011, 0.0012, 0.0013, 0.0014, 0.0015, 0.0016, 0.0017, 0.0018, 0.0019, 0.002, 0.0021, 0.0022, 0.0023, 0.0024, 0.0025, 0.0026, 0.0027, 0.0028, 0.0029, 0.003, 0.0031, 0.0032, 0.0033, 0.0034, 0.0035, 0.0036, 0.0037, 0.0038, 0.0039, 0.004, 0.0041, 0.0042, 0.0043, 0.0044, 0.0045, 0.0046, 0.0047, 0.0048, 0.0049, 0.005, 0.0051, 0.0052, 0.0053, 0.0054, 0.0055, 0.0056, 0.0057, 0.0058, 0.0059, 0.006, 0.0061, 0.0062, 0.0063, 0.0064, 0.0065, 0.0066, 0.0067, 0.0068, 0.0069, 0.007, 0.0071, 0.0072, 0.0073, 0.0074, 0.0075, 0.0076, 0.0077, 0.0078, 0.0079, 0.008, 0.0081, 0.0082, 0.0083, 0.0084, 0.0085, 0.0086, 0.0087, 0.0088, 0.0089, 0.009, 0.0091, 0.0092, 0.0093, 0.0094, 0.0095, 0.0096, 0.0097, 0.0098, 0.0099, 0.01, 0.011, 0.012, 0.013, 0.014, 0.015, 0.016, 0.017, 0.018, 0.019, 0.02, 0.021, 0.022, 0.023, 0.024, 0.025, 0.026, 0.027, 0.028, 0.029, 0.03, 0.031, 0.032, 0.033, 0.034, 0.035, 0.036, 0.037, 0.038, 0.039, 0.04, 0.041, 0.042, 0.043, 0.044, 0.045, 0.046, 0.047, 0.048, 0.049, 0.05, 0.051, 0.052, 0.053, 0.054, 0.055, 0.056, 0.057, 0.058, 0.059, 0.06, 0.061, 0.062, 0.063, 0.064, 0.065, 0.066, 0.067, 0.068, 0.069, 0.07, 0.071, 0.072, 0.073, 0.074, 0.075, 0.076, 0.077, 0.078, 0.079, 0.08, 0.081, 0.082, 0.083, 0.084, 0.085, 0.086, 0.087, 0.088, 0.089, 0.09, 0.091, 0.092, 0.093, 0.094, 0.095, 0.096, 0.097, 0.098, 0.099, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, and 1. In some preferred embodiments according to the fifth aspect, the concentration of DHP and/or at least one of its salts in the medium is about n mM, and n is selected from the list consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100. In this particular context, the term "about" refers to +/- 20 %.

According to a sixth aspect of the current invention, there is provided a eukaryotic cell, wherein at least one polynucleotide according to the third aspect has been introduced. In some embodiments according to the sixth aspect, the eukaryotic cell according to the sixth aspect is a cell for large scale protein production. In some embodiments according to the sixth aspect, the eukaryotic cells is a NS0 myeloma cell, COS cell, HEK293 cell, HEK293T cell, HEK293-EBNA cell, HEK293E cell, HEK293-6E cell, HEK293-Freestyle cell, HKB1 1 cell, BHK21 cell, Expi293F cell, 293EBNALT75 cell, CAP cell, CAP-T cell, EB66 cell, SP2 cell, or a cell from another mammalian cell line, wherein said cell or its ancestor has been engineered to obtain a proline auxotrophic mammalian host cell. Suitable methods to engineer the cells in order to introduce a proline auxotrophy include methods for knockout of P5CS and are well known in the art.

In some embodiments according to the sixth aspect, the eukaryotic cell according to the sixth aspect is a cell that can be cultured in suspension culture. In some preferred embodiments according to the sixth aspect, the eukaryotic cell is a Chinese Hamster Ovary (CHO) cell, such as a CHO-K1, CHO-S, CHO-K1SV, CHO Freestyle, CHOEBNALT85, CHOS-XE, CHO-DG44, CHO-DXB11 or CHO-3E7 cell.

In some preferred embodiments according to the sixth aspect, the vector or polynucleotide according to the third aspect has been introduced by transfection or viral transduction. In some preferred embodiments according to the sixth aspect, the vector or polynucleotide according to the third aspect has been introduced by electroporation, nucleofection, calcium-phosphate precipitation, lipofection, polycation-based transfection such as polyethlylenimine (PEI)-based transfection or DEAE-dextran transfection or any other technique known in the art. In some preferred embodiments according to the sixth aspect, the vector or polynucleotide according to the third aspect has been introduced by nucleofection.

In a highly preferred embodiment, the eukaryotic cell according to the sixth aspect is proline prototroph. In a preferred embodiment introducing an expression vector or polynucleotide according to the third aspect results in the expression of amounts of functional P5CS that are sufficient to render the eukaryotic cell proline prototroph. In a highly preferred embodiment, there is provided a eukaryotic cell according to the sixth aspect, wherein the expression of functional P5CS renders the cell proline prototroph. In a highly preferred embodiment, there is provided a eukaryotic cell according to the sixth aspect, wherein the expression of functional P5CS renders the cell proline prototroph and wherein the thereby obtained cell population after expansion yields for at least one of the at least one POI a production profile > n mg/L obtained as described for Example 9, wherein n is selected from the list consisting of 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, 500, 505, 510, 515, 520, 525, 530, 535, 540, 545, 550, 555, 560, 565 570, 575, 580, 585, 590, 595, 600, 605, 610, 615, 620, 625, 630, 635, 640, 645, 650, 655, 660, 665, 670, 675, 680, 685, 690, 695, 700, 705, 710, 715, 720, 725, 730, 735, 740, 745, 750, 755, 760, 765, 770, 775, 780, 785, 790, 795, 800, 805, 810, 815, 820, 825, 830, 835, 840, 845, 850, 855, 860, 865, 870, 875, 880, 885, 890, 895, 900, 905, 910, 915, 920, 925, 930, 935, 940, 945, 950, 955, 960, 965, 970, 975, 980, 985, 990, 995, 1000. In this particular context, the term "about" refers to +/- 20 %.

### EXAMPLES

### Example 1

### General design of the Selection System

To efficiently test the impact of different procedures on improving selection stringency, experimental testing in stable cell populations was performed. As test molecules, enhanced green fluorescent protein (GFP) as well as a human IgG1 were chosen. CHO-K1 cells adapted to growth in suspension in defined medium were obtained from SAFC and cultivated in EX-CELL CD CHO Fusion medium (SAFC 14365C). Custom-made EX-CELL CD CHO Fusion medium without L-Proline (Pro) and without L-Ornithine (Orn) ("proline-free medium") was obtained from SAFC. The expression vector system was based on modified versions of "CET 1019 AS-puro-SceI" and "CET 1019 AD-puro-SceI" for GFP and human IgG1, respectively, from Merck-Millipore, containing the Puromycin selection marker (Puromycin N-acetyl-transferase) known to exert a high selection stringency. To minimize variations in cell populations derived from using different transfections or different vectors, the first series of experiments were conducted using vectors into which a P5CS expression cassette was cloned upstream of a UCOE element, in addition to the Puromycin selection marker present downstream of a gene of interest. This way, it was possible to pool different transfections using the same vector to obtain a common starting point, and split the mixture according to different selection conditions, minimizing any variations that are not due to selection conditions themselves. Expression of long and short isoform of murine P5CS (Protein: Delta-1-pyrroline-5-carboxylate synthase; Gene: Aldh18a1; UniProtKB accession number: Q9Z110-1 & Q9Z110-2) was driven by a SV40 promoter. A SV40 polyadenylation sequence was used at the 3' end of the gene. A schematic representation of a vector containing relevant elements is shown in Fig. 2. More details can be found in Table 1. Typically, CHO cells were cultivated in EX-CELL CD CHO Fusion medium containing 6 mM GlutaMAX (Thermo Fisher, 35050-38) ("CD-Fusion medium") at 37 °C with 5% CO2. Cells were grown to logarithmic phase under shaking conditions for transfections. 1e7 cells/cuvette were transfected using nucleofection (Amaxa Nucleofector II, following the manufacturer's recommendations) with 5 µg of linearized DNA. After transfection, cells were taken up in 1 ml medium and transferred into 1 well of a 6 well microtiter plate (MTP) already containing 1 ml medium. The cells were incubated for 24 h without shaking, after which 9 ml medium were added and cells were washed (either in standard or proline-free medium). Multiple transfections were pooled, if applicable. Cells were adjusted to 2e5 viable cells/ml and 10 ml were transferred to T-25 flasks. Reference cell populations were selected using 10 µg/ml Puromycin in CD-Fusion medium. For P5CS-based selection, EX-CELL CD CHO Fusion medium (custom-made, SAFC) containing 6 mM GlutaMAX without proline and without ornithine was used ("proline-free" or "CD-Fusion w/o Pro" medium), with or without additional selection pressure.

Cell viability of the different populations was monitored over time. As soon as cell populations appeared to recover from selection pressure, cells were transferred to Shake Flask or TubeSpins for expansion in suspension under shaking conditions. Subsequently, stable cell populations transfected with IgG were seeded at 2e5 viable cells/ml for batch fermentation without selection pressure (i.e. standard CD-Fusion medium containing proline and no other selective agents). The cells were cultivated for 5-10 days, viable cell densities and productivity were monitored over time. IgG titers were determined using Poros A-HPLC method. Typically, final batch titers were recorded at a time point at which majority of cultures had around 70% viability. Stable cell populations transfected with GFP vector were subjected to analysis by flow cytometry using Guava easyCyte 8HT Benchtop Flow Cytometer (Millipore). 250 µl of cell culture were transferred to 96 MTP and washed with PBS. Dead cells were excluded from analysis using propidium iodide (PI). Since the dynamic range of 5 orders of magnitude of the instrument was insufficient to measure both, negative cells and transfected cells at the same time, fluorescence of GFP was not recorded using green channel (being the normal procedure), but instead using yellow channel of the instrument to dampen the fluorescent signal derived from GFP. The Median Fluorescent Intensity was determined as well as the percentage of highly fluorescing cells using an experiment-specific fluorescence threshold.

**Table 1: Table of expression vectors used for the examples including information on gene of interest (GOI), selection markers as well as positioning of selection marker expression cassettes.**

| **Vector Name** | **Gene of interest (GOI)** | **Selection marker expression cassette upstream of GOI** | **Selection marker expression cassette downstream of GOI** |
|---|---|---|---|
| pJF1556 | GFP | P5CS (long) | Puromycin N-acetyl-transferase |
| pJF1555 | GFP | P5CS (short) | Puromycin N-acetyl-transferase |
| pJF1577 | human IgG1 | P5CS (long) | Puromycin N-acetyl-transferase |
| pJF1576 | human IgG1 | P5CS (short) | Puromycin N-acetyl-transferase |
| pJF1979 | human IgG1 | - | Puromycin N-acetyl-transferase |
| pJF1969 | human IgG1 | - | P5CS (short) |
| pJF1970 | human IgG1 | - | P5CS (long) |
| pJF2053 | GFP | - | Puromycin N-acetyl-transferase |
| pJF2222 | GFP | Puromycin N-acetyl-transferase | - |
| pJF2055 | GFP | - | P5CS (long) |
| pJF2303 | GFP | P5CS (long) | - |
| pJF2460 | GFP | P5CS (long) driven by Delta3SV40E Promoter | - |

### Example 2

### A2C increases stringency but without increasing expression performance

US20140073007 reports that selection based on P5CS in proline-free medium is just comparable to that of antibiotic selection but not superior. It was therefore assumed, that sole selection under proline-free condition would not be superior to selection with Puromycin. In a first attempt, it was tested, whether A2C (L-Azetidine-2-carboxylic acid) (Sigma A0760) added to proline-free medium, would lead to an increase in stringency for the selection system. A2C has been previously reported to increase selective pressure (Baich, Somatic Cell Genet. 1977 Sep;3(5):529-38). In a variation of this setting, a combination of proline-free selection, puromyin and A2C was evaluated. For this experiment, vector pJF1556 was used containing both Puromycin selection marker as well as long isoform of mouse P5CS. Selection under Puromycin (Sigma P9620) served as reference condition. As control, a cell population was selected with Puromycin in proline-free medium, into which 0.25 mM proline was added back. In addition, mock transfections without DNA served as further controls. Table 2 shows viabilities determined on day 13 post-transfection. As evident from Table 2, cell populations selected with Puromycin only or selected in proline-free medium recovered with similar kinetics (#1, #2, #3). This was similar for a double selection with proline-free medium and Puromycin (#7). However, addition of A2C had a significant dose dependent effect on viability and recovery kinetics, indicative of increased selection pressure/burden on the cells. In fact, cultures #6, #9, and #10 failed to entirely recover even after extended cultivation. Stable cell populations were subsequently expanded under basic selection conditions (i.e. either standard CD Fusion medium containing Puromycin or proline-free CD Fusion medium with Puromycin added as appropriate, but no additional A2C was added). Table 3 summarizes the results obtained using flow cytometric analysis of cell populations on day 20 post-transfection. As evident from Table 3, selection using proline-free conditions using P5CS were not superior to selections based on Puromycin (#1,#2 vs #3). None of the conditions employing combinations of proline-free medium, Puromycin and A2C resulted in substantially (>1.42-fold) better levels of MFI or percentages of high fluorescent cells (#4 - #7). In summary, these results confirmed P5CS selection using proline-free medium being not superior to selection using an antibiotic marker. The results also indicate that treatment with A2C is able to increase selective burden on the cells (indicated by lower viabilities) but, however, this does not translate in superior expression performance.

**Table 2: Cell viabilities determined on day 13 post-transfection**

| **Vector** | # | **Selection Condition** | **Viability on day 13 post-transfection [%]** |
|---|---|---|---|
| pJF1556 | 1 | CD Fusion+ 10 µg/ml Puromycin | 81 |
| | 2 | CD-Fusion w/o Pro + 0.25 mM Pro + 10 µg/ml Puromycin | 90 |
| | 3 | CD-Fusion w/o Pro | 85 |
| | 4 | CD-Fusion w/o Pro + 0.1 mM A2C | 85 |
| | 5 | CD-Fusion w/o Pro + 0.25 mM A2C | 70 |
| | 6 | CD-Fusion w/o Pro + 0.5 mM A2C | 21 |
| | 7 | CD-Fusion w/o Pro + 10 µg/ml Puromycin | 92 |
| | 8 | CD-Fusion w/o Pro + 0.1 mM A2C + 10 µg/ml Puromycin | 62 |
| | 9 | CD-Fusion w/o Pro + 0.25 mM A2C + 10 µg/ml Puromycin | 22 |
| | 10 | CD-Fusion w/o Pro + 0.5 mM A2C + 10 µg/ml Puromycin | 21 |
| MOCK | 11 | CD Fusion +10 µg/ml Puromycin | 9 |
| | 12 | CD-Fusion w/o Pro +0.25mM Pro + 10 µg/ml Puromycin | 13 |
| | 13 | CD-Fusion w/o Pro | 12 |
| | 14 | CD-Fusion w/o Pro + 10 µg/ml Puromycin | 14 |
| | 15 | CD-Fusion w/o Pro +0.5 mM A2C +10 µg/ml Puromycin | 15 |

**Table 3: Flow cytometric analysis using Guava easyCyte 8HT Benchtop Flow Cytometer. Yellow Fluorescence Channel was used due to the high expression of GFP. Median Fluorescence Intensity (MFI) as well as frequency of high fluorescent cells were determined.**

| **Vector** | # | **Selection Condition** | **Median Fluorescence Intensity (MFI) (yellow)** | **Frequency of high fluorescing cells [%]** |
|---|---|---|---|---|
| pJF1556 | 1 | CD Fusion+ 10 µg/ml Puromycin | 7827 | 6.9 |
| | 2 | CD-Fusion w/o Pro+0.25 mM Pro+ 10 µg/ml Puromycin | 8783 | 9.6 |
| | 3 | CD-Fusion w/o Pro | 5020 | 4.1 |
| | 4 | CD-Fusion w/o Pro + 0.1 mM A2C | 5743 | 7.1 |
| | 5 | CD-Fusion w/o Pro + 0.25 mM A2C | 7578 | 7.1 |
| | 6 | CD-Fusion w/o Pro + 10 µg/ml Puromycin | 9370 | 8.7 |
| | 7 | CD-Fusion w/o Pro + 0.1 mM A2C + 10 µg/ml Puromycin | 9448 | 13.6 |
| - | | CHO-K1 parental | 4 | 0.1 |

### Example 3

### Ornithine increases stringency but without increasing expression performance

Ornithine has been reported to exert feedback inhibition on short isoform of P5CS (Hu et al., J Biol Chem. 1999 Mar 5;274(10):6754-62). Therefore, it was tested whether using selection under proline-free conditions in combination with ornithine and short isoform of P5CS could create conditions under which increased selective burden would translate into superior expression performance. From a mechanistic perspective this might be plausible, since L-Ornithine directly inhibits P5CS, i.e. the enzyme that is used for selection, in contrast to A2C, which may act in a more indirect way (Wasmuth and Caskey, Cell. 1976 May;8(1):71-7). For this experiment, the vector pJF1555 was used containing both Puromycin selection marker as well as short isoform of mouse P5CS. Selection under Puromycin served as reference condition. As control, a cell population was selected with Puromycin in proline-free medium into which 0.25 mM proline was added back. In addition, mock transfections without DNA served as further controls. Table 4 shows viabilities determined on day 14 post-transfection. As evident from Table 4, cell populations selected with Puromycin only or selected in proline-free medium recovered with similar kinetics (#1, #2, #3). This was similar for a double selection with proline-free medium and Puromycin (#7). However, addition of Ornithine (Orn) (Sigma 06503) had a significant effect on viability and recovery kinetics, indicative of increased selection pressure/burden on the cells (see #1,#2,#3 vs. #6 and #10). Interestingly, mock-transfected cultures selected under proline-free conditions with additional Orn (#15) seemed to have an increased level of background survival. For expansion, cells were kept in same medium with basic selection pressure, i.e. without additional Ornithine. Table 5 summarizes the results obtained using flow cytometric analysis of cell populations on day 15 post-transfection. As evident from Table 5, selections using proline-free conditions using P5CS are non-superior to selections based on Puromycin (#1, #2 vs #3). None of the conditions employing combinations of proline-free medium, Puromycin and Orn result in significantly better levels of MFI or percentages of high fluorescent cells (#4 - #10). The results indicate that treatment with Orn is able to increase selective burden on the cells (indicated by lower viabilities) but, however, this does not translate in superior expression performance. On the contrary, cell populations selected with proline-free medium containing additional 5 mM Orn (#6) displayed a significantly decreased expression performance compared to either cells selected by Puromycin or by selection in proline-free medium. One explanation for this result is that Orn - apart from inhibiting P5CS by feedback inhibition - triggers resistance by alternative pathways. For instance, upregulation/reactivation of ornithine aminotransferase (OAT) could actually be harnessed by cells if Orn is supplied as well. This explanation is consistent with the observation of an increased level of background survival in mock-transfected cultures selected under proline-free conditions but with additional Orn in medium. In summary, these results confirm earlier findings in literature of P5CS selection using proline-free medium being non-superior to selection using an antibiotic marker. In this example this was confirmed using short isoform of mouse P5CS. Finally, use of ornithine has no beneficial impact on expression performance (rather on the contrary) despite apparently increased selective pressure for the cells.

**Table 4: Cell viabilities determined on day 14 post-transfection.**

| **Vector** | # | **Selection Condition** | **Viability on day 14 post-transfection [%]** |
|---|---|---|---|
| pJF1555 | 1 | CD Fusion + 10 µg/ml Puromycin | 75 |
| | 2 | CD-Fusion w/o Pro + 0.25 mM Pro + 10 µg/ml Puromycin | 79 |
| | 3 | CD-Fusion w/o Pro | 82 |
| | 4 | CD-Fusion w/o Pro + 0.2 mM Orn | 83 |
| | 5 | CD-Fusion w/o Pro + 1 mM Orn | 87 |
| | 6 | CD-Fusion w/o Pro + 5 mM Orn | 51 |
| | 7 | CD-Fusion w/o Pro + 10 µg/ml Puromycin | 79 |
| | 8 | CD-Fusion w/o Pro + 0.2 mM Orn + 10 µg/ml | 73 |
| | | Puromycin | |
| | 9 | CD-Fusion w/o Pro + 1 mM Orn + 10 µg/ml Puromycin | 75 |
| | 10 | CD-Fusion w/o Pro + 5 mM Orn + 10 µg/ml Puromycin | 13 |
| MOCK | 11 | CD Fusion + 10 µg/ml Puromycin | 19 |
| | 12 | CD-Fusion w/o Pro + 0.25mM Pro + 10 µg/ml Puromycin | 20 |
| | 13 | CD-Fusion w/o Pro | 28 |
| | 14 | CD-Fusion w/o Pro + 10 µg/ml Puromycin | 23 |
| | 15 | CD-Fusion w/o Pro + 5 mM Orn | 43 |

**Table 5: Flow cytometric analysis using Guava easyCyte 8HT Benchtop Flow Cytometer. Yellow Fluorescence Channel was used due to the high expression of GFP. Median Fluorescence Intensity (MFI) as well as frequency of high fluorescent cells were determined.**

| **Vector** | # | **Selection Condition** | **Median Fluorescence Intensity (MFI) (yellow)** | **Frequency of high fluorescing cells [%]** |
|---|---|---|---|---|
| pJF1555 | 1 | CD Fusion+ 10 µg/ml Puromycin | 5072 | 5.1 |
| | 2 | CD-Fusion w/o Pro + 0.25 mM Pro + 10 µg/ml Puromycin | 4394 | 4.6 |
| | 3 | CD-Fusion w/o Pro | 3228 | 2.0 |
| | 4 | CD-Fusion w/o Pro + 0.2 mM Orn | 3337 | 2.4 |
| | 5 | CD-Fusion w/o Pro + 1 mM Orn | 3121 | 2.4 |
| | 6 | CD-Fusion w/o Pro + 5 mM Orn | 1435 | 0.5 |
| | 7 | CD-Fusion w/o Pro + 10 µg/ml Puromycin | 5102 | 5.1 |
| | 8 | CD-Fusion w/o Pro + 0.2 mM Orn + 10 µg/ml Puromycin | 4665 | 4.1 |
| | 9 | CD-Fusion w/o Pro + 1 mM Orn + 10 µg/ml Puromycin | 3739 | 2.8 |
| | 10 | CD-Fusion w/o Pro + 5 mM Orn + 10 µg/ml Puromycin | 4103 | 6.1 |
| - | 11 | CHO-K1 parental | 4 | 0.1 |

### Example 4

### Different types of POI do not influence the effect of A2C or Orn

Neither addition of A2C nor addition of ornithine resulted in the desired outcome, e.g. an increase in selection pressure with a concomitant increase in recombinant protein expression (Example 2 and Example 3). As GFP is released into the cytoplasm, a human IgG1 was tested as an example of a secretory protein to rule out that the results observed were due to the model protein used. For this experiment, the vectors pJF1577 (long isoform) and pJF1576 (short isoform) were used containing both Puromycin selection marker as well as long and short isoforms of mouse P5CS, respectively. Selection under Puromycin served as reference condition. As control, a cell population was selected with Puromycin in proline-free medium into which 0.25 mM proline was added back. Table 6 shows batch cultivation titers of human IgG1 measured by Poros A-HPLC on day 7 under non-selecting conditions (i.e. standard CD-Fusion medium containing proline and no additional selective agents like A2C, Orn, or Puromycin). These results confirmed earlier findings in literature of P5CS selection using proline-free medium being non-superior to selection using an antibiotic marker. In fact, none of the compounds known to exert selective pressure (A2C, Orn, Puromycin and combinations) resulted in significantly improved production titer. On the contrary, especially selection with 5 mM Orn (#16) resulted in very poor productivities despite very comparable viable cell density kinetics of this culture. These results also exclude the possibility that mediocre performance of P5CS selection under proline-free conditions observed even with additional selection pressure induced using A2C, Orn or double selection with Puromycin is due to differences between cytoplasmic and secreted proteins to be expressed.

**Table 6: Batch cultivation titers in shake flasks of human IgG1 measured by Poros A-HPLC on day 7 under non-selecting conditions.**

| **Vector** | # | **Selection Condition** | **Batch titer day 7 [mg/L]** |
|---|---|---|---|
| pJF1577 | 1 | CD Fusion+ 10 µg/ml Puromycin | 174 |
| | 2 | CD-Fusion w/o Pro + 0.25 mM Pro + 10 µg/ml Puromycin | 127 |
| | 3 | CD-Fusion w/o Pro | 124 |
| | 4 | CD-Fusion w/o Pro + 0.1 mM A2C | 135 |
| | 5 | CD-Fusion w/o Pro + 0.25 mM A2C | 166 |
| | 6 | CD-Fusion w/o Pro + 0.5 mM A2C | 166 |
| | 7 | CD-Fusion w/o Pro + 10 µg/ml Puromycin | 154 |
| | 8 | CD-Fusion w/o Pro + 0.1 mM A2C + 10 µg/ml Puromycin | 82 |
| | 9 | CD-Fusion w/o Pro + 0.25 mM A2C + 10 µg/ml Puromycin | 145 |
| | 10 | CD-Fusion w/o Pro + 0.5 mM A2C + 10 µg/ml Puromycin | 34 |
| pJF1576 | 11 | CD Fusion + 10 µg/ml Puromycin | 145 |
| | 12 | CD-Fusion w/o Pro+0.25 mM Pro+ 10 µg/ml Puromycin | 164 |
| | 13 | CD-Fusion w/o Pro | 125 |
| | 14 | CD-Fusion w/o Pro + 0.2 mM Orn | 120 |
| | 15 | CD-Fusion w/o Pro + 1 mM Orn | 135 |
| | 16 | CD-Fusion w/o Pro + 5 mM Orn | 9 |
| | 17 | CD-Fusion w/o Pro + 10 µg/ml Puromycin | 198 |
| | 18 | CD-Fusion w/o Pro + 0.2 mM Orn + 10 µg/ml Puromycin | 173 |
| | 19 | CD-Fusion w/o Pro + 1 mM Orn + 10 µg/ml Puromycin | 159 |
| | 20 | CD-Fusion w/o Pro + 5 mM Orn + 10 µg/ml Puromycin | 38 |

### Example 5

### Evaluation of various compounds for increased stringency and POI expression yields

To identify a compound which is not only increasing selective pressure on cells selected with P5CS selection marker in proline-free medium but which also leads to a concomitant increase of expression performance, various compounds were tested (Table 7). The strongest evidence available for a direct inhibition of P5CS is for proline and for ornithine. Both of these compounds have previously been shown to inhibit P5CS by feedback inhibition. Also, A2C has been used in the past to study proline biosynthesis in CHO cells, although it has never been shown that it can act on P5CS directly. The ability of ornithine to fulfil the desired profile for selection purposes was probed using a fine titration. It was conceivable that ornithine may lead to a counterproductive effect at high concentrations, but still could be beneficial at lower concentrations for high stringency selection of high expression cell lines. Other compounds like trans-4-Hydroxy-L-Proline, cis-4-Hydroxy-L-Proline, Thiazolidine-2-carboxylic acid, and DHP have been implicated much less specifically in inhibition of proline synthesis in e.g. Drosophila or as inhibitors of proline incorporation in *E. coli* into proteins. None of these have been shown to act on P5CS directly. In fact, DHP has been shown to be a substrate for P5C reductase, to be an inhibitor of prolyl hydroxylase and an inhibitor of Proline Oxidase instead. Moreover, Wasmuth and Caskey (Wasmuth and Caskey, Life Sci. 1978 May 1;22(17):1469-80) claim that DHP has no effect on the synthesis of proline from glutamic acid. Vector pJF1555 was used containing both Puromycin selection marker as well as short isoform of mouse P5CS. As reference, a cell population was selected with Puromycin in proline-free medium into which 0.25 mM proline was added back. Table 8 shows viabilities determined on day 12 post transfection. As evident from Table 8, cell populations selected in proline-free medium recovered with similar kinetics as compared to selection with Puromycin (#1 vs #2). There was no increased selection pressure observable for trans-4-Hydroxy-L-Proline (Sigma H5534) up to 10 mM, either indicating no effect on or no efficient uptake into the cells. In contrast, recovery kinetics were affected by 1 mM cis-4-HydroxyL-Proline (Sigma H16379) and higher. Similarly, at 0.5 - 1 mM Thiazolidine-2-carboxylic acid (Sigma 467995) started to affect cell recovery. An effect of L-Ornithine on recovery kinetics was observable with a concentration of 2 mM and higher. In summary, cis-4-Hydroxy-L-Proline, Thiazolidine-2-carboxylic acid, and L-Ornithine were able to affect recovery kinetics indicative of increased selection pressure. In this experiment, stable cell populations were expanded under non-selective conditions (i.e. standard CD Fusion medium without Puromycin, but containing proline). Table 9 summarizes the results obtained using flow cytometric analysis of cell populations on day 15 post-transfection. As evident from Table 9, selection using proline-free conditions using P5CS are non-superior to selections based on Puromycin. None of the conditions employing combinations of proline-free medium and trans-4-Hydroxy-L-Proline, cis-4-Hydroxy-L-Proline, Thiazolidine-2-carboxylic or L-Ornithine resulted in significantly higher percentages of high fluorescent cells. On the contrary, even though recovery kinetics were affected by cis-4-HydroxyL-Proline, Thiazolidine-2-carboxylic and L-Ornithine, this rather resulted in a decreased percentage of highly fluorescent cells.

**Table 7: Proline-related compounds with a reported connection to proline biosynthesis pathway.**

| **Compound** | **Structure** | **Literature Report** | **Reference** |
|---|---|---|---|
| L-Proline | 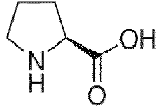 | Feedback inhibition of P5CS by end product | Pérez-Arrellano et al., Protein Sci. 2010 Mar; 19(3):372-82 |
| L-Ornithine | 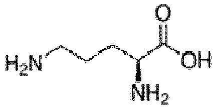 | Inhibitor of P5CS short isoform by feedback inhibition (isoforms are regulated differentially) | Hu et al., J Biol Chem. 1999 Mar 5; 274(10): 6754-62 |
| L-Azetidine-2-carboxylic acid | 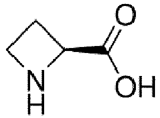 | Incorporated into proteins in place of proline & inhibits some enzyme between glutamate and P5CS | Wasmuth and Caskey, Cell 1976 May;8(1):71-7. |
| trans-4-HydroxyL-Proline | 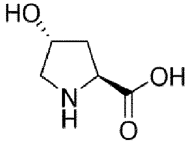 | non-proteinogenic amino acid formed by the post-translational hydroxylation of proline. Hydroxyproline is a major component of collagen, "hydroxy-proline" has been described to inhibit proline synthesis in *Drosophila* larvae | Rapport et al., Insect Biochem. 1979, 9:19-22 |
| cis-4-Hydroxy-L-Proline | 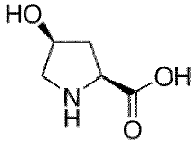 | cis-4-Hydroxy-L-Proline (CHP) is a proline analog that inhibits collagen synthesis and has been used as an anticancer compound. "hydroxy-proline" has been described to inhibit proline synthesis in *Drosophila* larvae | |
| Thiazolidine-2-carboxylic acid | 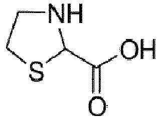 | is activated and transferred to tRNAPro by *Escherichia coli* and rat liver aminoacyl-tRNA synthetases, and inhibits proline incorporation into polypeptides in protein synthesizing systems from *E. coli,* rat liver or rabbit reticulocytes. In mammalian systems beta-thiaproline inhibits also leucine incorporation | Elthon and Stewart, Plant Physiol. 1984 Feb;74(2):213-8 |
| | | | (on Thiazolidine-4-carboxylic acid) |
| 3,4-Dehydro-L-Proline | 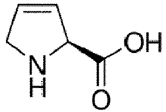 | Substrate for P5C Reductase (P5CR); | Meng et al., Protein Expr Purif. 2006 Sep;49(1):83- 7 |
| | | selective inhibitor of prolyl hydroxylase; competitive inhibitor of Proline Oxidase | Cooper and Varner, Plant Physiol. 1983 Oct;73(2):324-328 |
| | | | Pandhare et al., J Cell Biochem. 2009 Jul 1;107(4):759-68 |

**Table 8: Cell viabilities determined post-transfection on the day indicated**

| **Vector** | # | **Selection Condition** | **Viability on day 12 post-transfection [%]** |
|---|---|---|---|
| | 1 | CD-Fusion w/o Pro + 0.25 mM Pro + 10 µg/ml Puromycin | 83 |
| | 2 | CD-Fusion w/o Pro | 87 |
| | 3 | CD-Fusion w/o Pro + 5 mM trans-4-Hydroxy-L-Proline | 84 |
| | 4 | CD-Fusion w/o Pro + 10 mM trans-4-Hydroxy-L-Proline | 84 |
| | 5 | CD-Fusion w/o Pro + 0.25 mM cis-4-Hydroxy-L-Proline | 89 |
| | 6 | CD-Fusion w/o Pro + 0.5 mM cis-4-Hydroxy-L-Proline | 88 |
| | 7 | CD-Fusion w/o Pro + 1 mM cis-4-Hydroxy-L-Proline | 65 |
| | 8 | CD-Fusion w/o Pro + 2.5 mM cis-4-Hydroxy-L-Proline | 13 |
| | 9 | CD-Fusion w/o Pro + 5 mM cis-4-Hydroxy-L-Proline | 13 |
| | 10 | CD-Fusion w/o Pro + 10 mM cis-4-Hydroxy-L-Proline | 20 |
| | 11 | CD-Fusion w/o Pro + 0.1 mM Thiazolidine-2-carboxylic acid | 87 |
| pJF1555 | 12 | CD-Fusion w/o Pro + 0.5 mM Thiazolidine-2-carboxylic acid | 78 |
| | 13 | CD-Fusion w/o Pro + 1 mM Thiazolidine-2-carboxylic acid | 11 |
| | 14 | CD-Fusion w/o Pro + 2.5 mM Thiazolidine-2-carboxylic acid | 15 |
| | 15 | CD-Fusion w/o Pro + 10 mM Thiazolidine-2-carboxylic acid | 16 |
| | 16 | CD-Fusion w/o Pro + 0.25 mM Ornithine | 88 |
| | 17 | CD-Fusion w/o Pro + 0.5 mM Ornithine | 87 |
| | 18 | CD-Fusion w/o Pro + 1 mM Ornithine | 91 |
| | 19 | CD-Fusion w/o Pro + 2 mM Ornithine | 49 |
| | 20 | CD-Fusion w/o Pro + 3 mM Ornithine | 36 |
| | 21 | CD-Fusion w/o Pro + 4 mM Ornithine | 55 |
| | 22 | CD-Fusion w/o Pro + 5 mM Ornithine | 53 |
| MOCK | 23 | CD Fusion + 10 µg/ml Puromycin | 25 |
| | 24 | CD-Fusion w/o Pro + 0.25 mM Pro + 10 µg/ml Puromycin | 23 |
| | 25 | CD-Fusion w/o Pro | 32 |
| | 26 | CD-Fusion w/o Pro + 5 mM Orn | 45 |

**Table 9: Flow cytometric analysis using Guava easyCyte 8HT Benchtop Flow Cytometer. Yellow Fluorescence Channel was used due to the high expression of GFP. Frequency of high fluorescent cells were determined.**

| **Vector** | # | **Selection Condition** | **Frequency of high fluorescing cells [%]** |
|---|---|---|---|
| | 1 | CD-Fusion w/o Pro + 0.25 mM Pro + 10 µg/ml Puromycin | 4.3 |
| | 2 | CD-Fusion w/o Pro | 3.4 |
| | 3 | CD-Fusion w/o Pro + 5 mM trans-4-Hydroxy-L-Proline | 2.6 |
| | 4 | CD-Fusion w/o Pro + 10 mM trans-4-Hydroxy-L-Proline | 2.6 |
| | 5 | CD-Fusion w/o Pro + 0.25 mM cis-4-Hydroxy-L-Proline | 3.3 |
| | 6 | CD-Fusion w/o Pro + 0.5 mM cis-4-Hydroxy-L-Proline | 1.7 |
| | 7 | CD-Fusion w/o Pro + 1 mM cis-4-Hydroxy-L-Proline | not determined |
| | 8 | CD-Fusion w/o Pro + 2.5 mM cis-4-Hydroxy-L-Proline | 1.4 |
| | 9 | CD-Fusion w/o Pro + 5 mM cis-4-Hydroxy-L-Proline | 0.0 |
| pJF1555 | 10 | CD-Fusion w/o Pro + 10 mM cis-4-Hydroxy-L-Proline | 0.0 |
| | 11 | CD-Fusion w/o Pro + 0.1 mM Thiazolidine-2-carboxylic acid | 4.9 |
| | 12 | CD-Fusion w/o Pro + 0.5 mM Thiazolidine-2-carboxylic acid | 2.3 |
| | 13 | CD-Fusion w/o Pro + 1 mM Thiazolidine-2-carboxylic acid | 0.0 |
| | 14 | CD-Fusion w/o Pro + 2.5 mM Thiazolidine-2-carboxylic acid | 0.0 |
| | 15 | CD-Fusion w/o Pro + 10 mM Thiazolidine-2-carboxylic acid | 0.0 |
| | 16 | CD-Fusion w/o Pro + 0.25 mM Ornithine | 6.3 |
| | 17 | CD-Fusion w/o Pro + 0.5 mM Ornithine | 4.1 |
| | 18 | CD-Fusion w/o Pro + 1 mM Ornithine | 2.3 |
| | 19 | CD-Fusion w/o Pro + 2 mM Ornithine | 0.7 |
| | 20 | CD-Fusion w/o Pro + 3 mM Ornithine | 2.6 |
| | 21 | CD-Fusion w/o Pro + 4 mM Ornithine | 1.5 |
| | 22 | CD-Fusion w/o Pro + 5 mM Ornithine | 1.7 |

### Example 6

### DHP increases stringency and POI expression yields

Vector pJF1555 was used containing both Puromycin selection marker as well as short isoform of mouse P5CS. Selection under Puromycin served as reference condition. As control, a cell population was selected with Puromycin in proline-free medium into which 0.25 mM proline was added back. Table 10 shows viabilities determined on day 19 post-transfection. As evident from Table 10, cell populations selected in proline-free medium recovered with similar kinetics as to selection with Puromycin (#1, #2, #3). DHP slowed down the recovery of cell populations selected in proline-free medium, indicative of increased selection pressure (#4). In fact, cultures with 1 mM DHP and above did not recover in this experiment, even after extension of cultivation Table 11 summarizes the results obtained using flow cytometric analysis of cell populations on day 19 post-transfection. As is evident from Table 11, selection using proline-free conditions using P5CS are non-superior to selections based on Puromycin. Unexpectedly, and in contrast to the other compounds tested, addition of 0.2 mM DHP resulted in a 37 % increase in MFI as compared to Puromycin-selected population (#4 vs #1). Similarly, the frequency of high fluorescing cells was increased by 71%. According to these results, DHP thus shows the ability to increase selection stringency resulting in concomitant increase in expression performance.

**Table 10: Cell viabilities determined post-transfection.**

| **Vector** | # | **Selection Condition** | **Viability on day 19 post-transfection [%]** |
|---|---|---|---|
| pJF1555 | 1 | CD-Fusion + 10 µg/ml Puromycin | 49 |
| | 2 | CD-Fusion w/o Pro + 0.25 mM Pro + 10 µg/ml Puromycin | 48 |
| | 3 | CD-Fusion w/o Pro | 54 |
| | 4 | CD-Fusion w/o Pro + 0.2 mM DHP | 27 |
| | 5 | CD-Fusion w/o Pro + 1 mM DHP | 7 |
| | 6 | CD-Fusion w/o Pro + 5 mM DHP | 6 |
| | 7 | CD-Fusion w/o Pro + 10 mM DHP | 7 |

**Table 11: Flow cytometric analysis using Guava easyCyte 8HT Benchtop Flow Cytometer.**

| **Vector** | # | **Selection Condition** | **Median Fluorescence Intensity (MFI) (yellow)** | **Frequency of high fluorescing cells [%]** |
|---|---|---|---|---|
| pJF1555 | 1 | CD-Fusion + 10 µg/ml Puromycin | 13092 | 3.4 |
| | 2 | CD-Fusion w/o Pro + 0.25mM Pro + 10 µg/ml Puromycin | 12860 | 3.6 |
| | 3 | CD-Fusion w/o Pro | 11096 | 2.9 |
| | 4 | CD-Fusion w/o Pro + 0.2 mM DHP | 17880 | 5.8 |
| - | 5 | CHO-K1 parental | 6 | 0.1 |

### Example 7

### Optimization of the concentration of DHP

To follow up on the initial observation described for Example 6, a fine titration of DHP levels was performed to optimize the concentration levels for DHP. For this experiment the vector pJF1555 was used containing both Puromycin selection marker as well as short isoform of mouse P5CS. Selection under Puromycin served as reference condition. As control, a cell population was selected with Puromycin in proline-free medium into which 0.25 mM proline was added back. Table 12 shows viabilities determined on day 12 post transfection. As evident from Table 12, DHP slowed down the recovery of cell populations selected in proline-free medium in a dose-dependent manner, indicative of increased selection pressure (#1 - #3 vs #4 - #8). In fact, under these conditions cultures with 0.8 mM DHP did not recover in this experiment (#8), even after prolonged incubations. Stable cell populations were expanded under non-selective conditions (i.e. standard CD Fusion medium without Puromycin, but containing proline). Table 13 summarizes the results obtained using flow cytometric analysis of cell populations on day 19 post-transfection. As evident from Table 13, selection using proline-free conditions using P5CS are non-superior to selections based on Puromycin (#1,#2 vs #3). Surprisingly, addition of increasing amounts of DHP into selections in proline-free medium had a beneficial impact on expression performance (#1-#3 vs #4-#7). Using 0.4 mM DHP, the MFI of the stable cell population resulted in a 3-fold improvement. Similarly, the frequency of high fluorescing cells was increased by 8.7-12.6-fold. In contrast to A2C, cis-4-Hydroxy-L-proline, Thiazolidine-2-carboxylic or Ornithine, further inclusion of DHP into selection conditions not only led to a slower recovery kinetic of the stable population (indicative of increased selection pressure), but importantly also concomitantly to significant and substantial increase in expression performance.

**Table 12: Cell viabilities determined post-transfection on the day indicated.**

| **Vector** | # | **Selection Condition** | **Viability on day 12 post-transfection [%]** |
|---|---|---|---|
| pJF1555 | 1 | CD-Fusion + 10 µg/ml Puromycin | 86 |
| | 2 | CD-Fusion w/o Pro + 0.25 mM Pro + 10 µg/ml Puromycin | 63 |
| | 3 | CD-Fusion w/o Pro | 90 |
| | 4 | CD-Fusion w/o Pro + 0.05 mM DHP | 83 |
| | 5 | CD-Fusion w/o Pro + 0.1 mM DHP | 53 |
| | 6 | CD-Fusion w/o Pro + 0.2 mM DHP | 40 |
| | 7 | CD-Fusion w/o Pro + 0.4 mM DHP | 20 |
| | 8 | CD-Fusion w/o Pro + 0.8 mM DHP | 13 |

**Table 13: Flow cytometric analysis using Guava easyCyte 8HT Benchtop Flow Cytometer. Yellow Fluorescence Channel was used due to the high expression of GFP. Median Fluorescence Intensity (MFI) as well as frequency of high fluorescent cells were determined.**

| **Vector** | # | **Selection Condition** | **Median Fluorescence Intensity (MFI) (yellow)** | **Frequency of high fluorescing cells [%]** |
|---|---|---|---|---|
| pJF1555 | 1 | CD-Fusion + 10 µg/ml Puromycin | 6167 | 1.0 |
| | 2 | CD-Fusion w/o Pro + 0.25 mM Pro + 10 µg/ml Puromycin | 6625 | 1.5 |
| | 3 | CD-Fusion w/o Pro | 5879 | 0.8 |
| | 4 | CD-Fusion w/o Pro + 0.05 mM DHP | 7599 | 1.7 |
| | 5 | CD-Fusion w/o Pro + 0.1 mM DHP | 14267 | 5.6 |
| | 6 | CD-Fusion w/o Pro + 0.2 mM DHP | 13748 | 5.8 |
| | 7 | CD-Fusion w/o Pro + 0.4 mM DHP | 19806 | 13.0 |
| pJF1576 (control) | 9 | CD-Fusion + 10 µg/ml Puromycin | 9 | 0.1 |

### Example 8

### Effect of DHP persists independent of the type of POI

To determine whether the observed effect of DHP occurs not only for an intracellular protein like GFP but also for a secreted protein, the impact of DHP on human IgG1 expression was evaluated. For this experiment, the vector pJF1576 was used containing the Puromycin selection marker as well as the short isoform of mouse P5CS. Selection under Puromycin served as reference condition. As control, a cell population was selected with Puromycin in proline-free medium into which 0.25 mM proline was added back. Table 14 shows viabilities determined on day 12 post transfection. As evident from Table 14, DHP slowed down the recovery of cell populations selected in proline-free medium in a dose-dependent manner, indicative of increased selection pressure (#3-#8). In fact, under the applied conditions cultures with 0.8 mM DHP did not recover in this experiment. Table 15, Table 16 and Table 17 show viable cell density, viability and productivity profiles of cell populations cultured in batch fermentation in shake flasks under non-selecting conditions (i.e. CD-Fusion medium containing proline but no additional selective agents like Puromycin or DHP) after inoculation at 2e5 viable cells/ml. The viable cell density and viability profile of the cultures are very comparable to each other. Unexpectedly, the productivity profile was beneficially affected by the presence of DHP during initial selection of the stable cell population under proline-free conditions. While a P5CS selection using proline-free medium is non-superior to selection using an antibiotic selection marker, the additional selective pressure by DHP resulted in significantly improved production titers. In fact, using 0.2 mM or 0.4 mM DHP as additional selection additives resulted in approximately 2-3-fold titer improvements (#1 - #3 vs #6 & #7).

**Table 14: Cell viabilities determined on day 12 post-transfection.**

| **Vector** | # | **Selection Condition** | **Viability on day 12 post-transfection [%]** |
|---|---|---|---|
| pJF1576 | 1 | CD-Fusion + 10 µg/ml Puromycin | 78 |
| | 2 | CD-Fusion w/o Pro + 0.25 mM Pro + 10 µg/ml Puromycin | 79 |
| | 3 | CD-Fusion w/o Pro | 92 |
| | 4 | CD-Fusion w/o Pro + 0.05 mM DHP | 91 |
| | 5 | CD-Fusion w/o Pro + 0.1 mM DHP | 74 |
| | 6 | CD-Fusion w/o Pro + 0.2 mM DHP | 49 |
| | 7 | CD-Fusion w/o Pro + 0.4 mM DHP | 28 |
| | 8 | CD-Fusion w/o Pro + 0.8 mM DHP | 23 |

**Table 15: Viable cell density profiles of cell populations cultured in batch fermentation in shake flasks under non-selecting conditions.**

| | **Viable cell density profile [VCD/ml]** | **Day after inoculation** | | | | |
|---|---|---|---|---|---|---|
| # | **Condition under which stable cell population was selected** | **0** | **2** | **4** | **7** | **9** |
| 1 | CD Fusion + 10 µg/ml Puromycin | 2,0E+05 | 7,7E+05 | 6,2E+06 | 1,2E+07 | 5,8E+06 |
| 2 | CD-Fusion w/o Pro + 0.25 mM Pro + 10 µg/ml Puromycin | 2,0E+05 | 8,3E+05 | 5,4E+06 | 1,0E+07 | 6,2E+06 |
| 3 | CD-Fusion w/o Pro | 2,0E+05 | 7,6E+05 | 5,2E+06 | 7,7E+06 | 5,3E+06 |
| 4 | CD-Fusion w/o Pro + 0.05 mM DHP | 2,0E+05 | 6,1E+05 | 5,3E+06 | 1,0E+07 | 2,5E+06 |
| 5 | CD-Fusion w/o Pro + 0.1 mM DHP | 2,0E+05 | 7,9E+05 | 6,5E+06 | 7,9E+06 | 6,4E+05 |
| 6 | CD-Fusion w/o Pro + 0.2 mM DHP | 2,0E+05 | 6,4E+05 | 5,2E+06 | 9,6E+06 | 2,2E+06 |
| 7 | CD-Fusion w/o Pro + 0.4 mM DHP | 2,0E+05 | 8,0E+05 | 6,1E+06 | 1,1E+07 | 3,1E+06 |

**Table 16: Viability profiles of cell populations cultured in batch fermentation in shake flasks under non-selecting conditions.**

| | **Viability profile [%]** | **Day after inoculation** | | | | |
|---|---|---|---|---|---|---|
| # | **Condition under which stable cell population was selected** | **0** | **2** | **4** | **7** | **9** |
| 1 | CD Fusion + 10 µg/ml Puromycin | 98.7 | 99.0 | 99.7 | 98.7 | 43.9 |
| 2 | CD-Fusion w/o Pro + 0.25 mM Pro + 10 µg/ml Puromycin | 99.0 | 99.1 | 99.8 | 97.8 | 58.1 |
| 3 | CD-Fusion w/o Pro | 98.9 | 98.8 | 99.7 | 99.2 | 51.3 |
| 4 | CD-Fusion w/o Pro + 0.05 mM DHP | 99.4 | 98.9 | 99.8 | 98.6 | 19.5 |
| 5 | CD-Fusion w/o Pro + 0.1 mM DHP | 99.0 | 99.3 | 99.7 | 95.6 | 5.1 |
| 6 | CD-Fusion w/o Pro + 0.2 mM DHP | 98.6 | 99.2 | 99.5 | 97.4 | 18.7 |
| 7 | CD-Fusion w/o Pro + 0.4 mM DHP | 96.3 | 99.3 | 99.6 | 98.1 | 21.7 |

**Table 17: Productivity profiles of cell populations cultured in batch fermentation in shake flasks under non-selecting conditions. Nd = not determined.**

| | **Productivity profile [mg/L]** | **Day after inoculation** | | | | |
|---|---|---|---|---|---|---|
| # | **Condition under which stable cell population was selected** | **0** | **2** | **4** | **7** | **9** |
| 1 | CD Fusion + 10 µg/ml Puromycin | nd | 6.9 | 15.1 | 43.3 | 61.5 |
| 2 | CD-Fusion w/o Pro + 0.25 mM Pro + 10 µg/ml Puromycin | nd | 11.2 | 24.1 | 60.6 | 92.5 |
| 3 | CD-Fusion w/o Pro | nd | 9.2 | 21.3 | 62.0 | 108.6 |
| 4 | CD-Fusion w/o Pro + 0.05 mM DHP | nd | 9.8 | 24.2 | 48.6 | 115.5 |
| 5 | CD-Fusion w/o Pro + 0.1 mM DHP | nd | 11.0 | 31.7 | 104.4 | 127.1 |
| 6 | CD-Fusion w/o Pro + 0.2 mM DHP | nd | 11.3 | 34.6 | 87.4 | 153.7 |
| 7 | CD-Fusion w/o Pro + 0.4 mM DHP | nd | 11.3 | 41.0 | 116.4 | 164.7 |

### Example 9

### Production of secretory POI by stable cell populations

To test whether stable cell populations would be able to produce significant amounts of secretory protein even under proline-free production conditions, the stable cell populations from Example 8 were used. The populations had been passaged under selective pressure, i.e. in CD-Fusion medium containing 10 µg/ml Puromycin for populations selected with Puromycin and CD-Fusion medium w/o proline in the case of populations initially selected under proline-free conditions and in the presence of DHP. No extra DHP was added during passaging. The same conditions used for passaging were now applied during batch fermentation in shake flasks under those selective conditions. Table 18, Table 19, and Table 20 show viable cell density, viability and productivity profiles of cell populations cultured in batch fermentation in shake flasks under selective conditions (i.e. CD-Fusion medium containing proline and additional Puromycin or proline-free CD-Fusion medium, respectively) after inoculation at 2e5 viable cells/ml. The viable cell density and viability profile of the cultures are very comparable to each other. Unexpectedly, the productivity profile was beneficially affected by the presence of DHP during initial selection of the stable cell population under proline-free conditions, even if the later batch cultivation was performed in proline-free medium (for a fair comparison, Puromycin selected cultures were cultivated in full CD-Fusion medium containing Puromycin). The additional selective pressures by DHP resulted in significantly improved production titers. In fact, using DHP as additional selection agent in initial selections resulted in approximately 2-3-fold titer improvements as compared to Puromycin-based selections of cell populations. These observations are consistent with the notion that proline synthesized by the cell upon expression of P5CS is enough to sustain high production titers of a secreted protein of choice without slowing down the growth and proliferation of the cells due to increased metabolic burden. This offers the potential to even run large scale production fermenters under selective conditions (i.e. without proline in medium). This would typically be avoided for cells which carry an antibiotic selection marker due to the complexities for manufacturing a biotherapeutic product comprising an antibiotic in large scale fermenters.

**Table 18: Viable cell density profiles of cell populations cultured in batch fermentation in shake flasks under selective conditions.**

| | **Viable cell density profile [VCD/ml]** | **Day after inoculation** | | | |
|---|---|---|---|---|---|
| # | **Condition under which stable cell population was selected** | **0** | **3** | **5** | **7** |
| 1 | CD Fusion + 10 µg/ml Puromycin | 2.0E+05 | 1.7E+06 | 7.1E+06 | 1.1E+07 |
| 2 | CD-Fusion w/o Pro | 2.0E+05 | 3.1E+06 | 7.9E+06 | 9.3E+06 |
| 3 | CD-Fusion w/o Pro + 0.05 mM DHP | 2.0E+05 | 5.6E+06 | 9.5E+06 | 4.6E+06 |
| 4 | CD-Fusion w/o Pro + 0.1 mM DHP | 2.0E+05 | 7.4E+06 | 1.2E+07 | 4.0E+06 |
| 5 | CD-Fusion w/o Pro + 0.2 mM DHP | 2.0E+05 | 2.4E+06 | 6.9E+06 | 9.1E+06 |
| 6 | CD-Fusion w/o Pro + 0.4 mM DHP | 2.0E+05 | 5.3E+06 | 1.1E+07 | 6.9E+06 |

**Table 19: Viability profiles of cell populations cultured in batch fermentation in shake flasks under selective conditions.**

| | **Viability profile [%]** | **Day after inoculation** | | | |
|---|---|---|---|---|---|
| # | **Condition under which stable cell population was selected** | **0** | **3** | **5** | **7** |
| 1 | CD Fusion + 10 µg/ml Puromycin | 92.8 | 98.4 | 99.1 | 97.7 |
| 2 | CD-Fusion w/o Pro | 98.4 | 98.8 | 98.6 | 92.9 |
| 3 | CD-Fusion w/o Pro + 0.05 mM DHP | 98.4 | 99.3 | 96.4 | 45.3 |
| 4 | CD-Fusion w/o Pro + 0.1 mM DHP | 97.5 | 99.4 | 97.5 | 32.2 |
| 5 | CD-Fusion w/o Pro + 0.2 mM DHP | 98.2 | 99.1 | 98.9 | 93.2 |
| 6 | CD-Fusion w/o Pro + 0.4 mM DHP | 99.7 | 99.3 | 96.1 | 56.1 |

**Table 20: Productivity profiles of cell populations cultured in batch fermentation in shake flasks under selective conditions.**

| | **Productivity profile [mg/L]** | **Day after inoculation** | | | |
|---|---|---|---|---|---|
| # | **Condition under which stable cell population was selected** | **0** | **2** | **4** | **7** |
| 1 | CD Fusion + 10 µg/ml Puromycin | nd | 10.9 | 27.6 | 60.5 |
| 2 | CD-Fusion w/o Pro | nd | 19.2 | 56.7 | 99.6 |
| 3 | CD-Fusion w/o Pro + 0.05 mM DHP | nd | 47.1 | 114.8 | 166.2 |
| 4 | CD-Fusion w/o Pro + 0.1 mM DHP | nd | 37.5 | 83.8 | 125.8 |
| 5 | CD-Fusion w/o Pro + 0.2 mM DHP | nd | 34.4 | 104.8 | 179.7 |
| 6 | CD-Fusion w/o Pro + 0.4 mM DHP | nd | 39.4 | 105.5 | 133.4 |

### Example 10

### Positioning of the P5CS expression cassette influences stringency

To test the performance of P5CS selection in combination with DHP in a production cell line development setting (i.e. only a single selection marker in expression vector), vectors with P5CS selection marker in place of Puromycin selection marker containing human IgG1 expression cassettes were generated and tested. For this experiment, the vector pJF1979 was taken as a reference, with Puromycin selection marker placed downstream of the human IgG1 expression cassette. For comparison, the vectors pJF1969 and pJF1970 were used, which contained short and long isoform of P5CS in place of Puromycin selection marker downstream of human IgG1 expression cassette. As control, vector pJF1576 from Example 8 was used, which contained both P5CS (upstream of human IgG1 expression cassette) and Puromycin (downstream of human IgG1 expression cassette) selection markers. Table 21 shows viabilities determined on day 21 post-transfection. As evident from Table 21, DHP slowed down the recovery of cell populations selected in proline-free medium, indicative of increased selection pressure (#3-#14). Culture #6 with 0.6 mM DHP showed poor growth and failed to fully recover. Quite unexpectedly, there was no cell population recovery even beyond day 21 post-transfection for cultures #7-#14 in this experiment, even at concentrations which supported growth for cells transfected with pJF1576. A possible explanation for this may be the positioning of the P5CS expression cassette (upstream of human IgG1 expression cassette in case of pJF1576 or downstream of human IgG1 expression cassette in case of pJF1969 and pJF1979). The positioning may influence the amount of P5CS produced and thereby modulate the optimal DHP concentration window useful for selective growth conditions. Nevertheless, stable cell populations that had recovered in this experiment were subjected to a fed-batch fermentation experiment in shake flasks for 14 days, as a fed-batch fermentation in principle resembles a later production process. The fed-batch was without pH control during cultivation and no additional glucose was fed apart from that present in feeding solutions. Table 22, Table 23 and Table 24 show viable cell density, viability and productivity profiles of cell populations cultured in fed-batch fermentation in shake flasks under non-selecting conditions (i.e. production medium containing proline and no additional selective agents like Puromycin or DHP) after inoculation at 3e5 viable cells/ml. All cultures reached high viable cell densities. Unexpectedly, the productivity profile was beneficially affected by the presence of DHP during initial selection of the stable cell population under proline-free conditions for cultures #3 and #4. In fact, using 0.2 mM or 0.4 mM DHP as additional selection resulted in approximately 1.6-2.1-fold titer improvements in comparison to Puromycin selected cultures. The observation that a culture selected with 0.5 mM DHP as additional selection agent resulted in lower titers may indicate that an optimal concentration level for DHP during selection phase exists. In summary, P5CS selection in proline-free medium in combination with DHP addition can lead to improved titers in a process resembling a production cell line development setting for an industrially relevant human IgG1.

**Table 21: Cell viabilities determined post-transfection on day 21.**

| **Vector** | # | **Selection Condition** | **Viability on day 21 post-transfection [%]** |
|---|---|---|---|
| pJF1579 | 1 | CD-Fusion + 10 µg/ml Puromycin | 98.8 |
| pJF1576 | 2 | CD-Fusion + 10 µg/ml Puromycin | 98.4 |
| | 3 | CD-Fusion w/o Pro + 0.2 mM DHP | 87.4 |
| | 4 | CD-Fusion w/o Pro + 0.4 mM DHP | 65.3 |
| | 5 | CD-Fusion w/o Pro + 0.5 mM DHP | 42.8 |
| | 6 | CD-Fusion w/o Pro + 0.6 mM DHP | 35.7 |
| pJF1969 | 7 | CD-Fusion w/o Pro + 0.2 mM DHP | 26.5 |
| | 8 | CD-Fusion w/o Pro + 0.4 mM DHP | 22.9 |
| | 9 | CD-Fusion w/o Pro + 0.5 mM DHP | 23.3 |
| | 10 | CD-Fusion w/o Pro + 0.6 mM DHP | 25.9 |
| pJF1970 | 11 | CD-Fusion w/o Pro + 0.2 mM DHP | 18.2 |
| | 12 | CD-Fusion w/o Pro + 0.4 mM DHP | 21.4 |
| | 13 | CD-Fusion w/o Pro + 0.5 mM DHP | 18.3 |
| | 14 | CD-Fusion w/o Pro + 0.6 mM DHP | 18.2 |

**Table 22: Viable cell density profiles of cell populations cultured in batch fermentation in shake flasks under non-selecting conditions.**

| | **Viable cell density profile [VCD/ml]** | **Day after inoculation** | | | | | | |
|---|---|---|---|---|---|---|---|---|
| **#** | **Condition under which stable cell population was selected** | **0** | **3** | **5** | **7** | **10** | **12** | **14** |
| 1 | CD-Fusion + 10 µg/ml Puromycin (pJF1579) | 3,0E +05 | 1,8E +06 | 5,6E +06 | 1,2E +07 | 1,5E +07 | 1,0E +07 | 9,7E +06 |
| 2 | CD-Fusion + 10 µg/ml Puromycin (pJF1576) | 3,0E +05 | 1,4E +06 | 4,8E +06 | 9,5E +06 | 1,2E +07 | 9,1E +06 | 7,4E +06 |
| 3 | CD-Fusion w/o Pro + 0.2 mM DHP (pJF1576) | 3,0E +05 | 2,1E +06 | 5,9E +06 | 1,3E +07 | 1,9E +07 | 1,8E +07 | 1,2E +07 |
| 4 | CD-Fusion w/o Pro + 0.4 mM DHP (pJF1576) | 3,0E +05 | 2,1E +06 | 7,6E +06 | 1,2E +07 | 6,9E +06 | 5,2E +06 | 5,6E +06 |
| 5 | CD-Fusion w/o Pro + 0.5 mM DHP (pJF1576) | 3,0E +05 | 1,7E +06 | 4,6E +06 | 7,6E +06 | 8,3E +06 | 5,5E +06 | 5,2E +06 |

**Table 23: Viability profiles of cell populations cultured in batch fermentation in shake flasks under non-selecting conditions.**

| | **Viability profile [%]** | **Day after inoculation** | | | | | | |
|---|---|---|---|---|---|---|---|---|
| **#** | **Condition under which stable cell population was selected** | **0** | **3** | **5** | **7** | **10** | **12** | **14** |
| 1 | CD-Fusion + 10 µg/ml Puromycin (pJF1579) | 99.8 | 99.8 | 99.9 | 99.2 | 97.9 | 97.5 | 92.7 |
| 2 | CD-Fusion + 10 µg/ml Puromycin (pJF1576) | 99.8 | 99.9 | 99.8 | 99.3 | 97.0 | 96.2 | 86.2 |
| 3 | CD-Fusion w/o Pro + 0.2 mM DHP (pJF1576) | 99.7 | 99.7 | 99.4 | 98.3 | 99.0 | 93.0 | 58.3 |
| 4 | CD-Fusion w/o Pro + 0.4 mM DHP (pJF1576) | 99.6 | 99.7 | 99.8 | 98.1 | 71.4 | 76.0 | 79.9 |
| 5 | CD-Fusion w/o Pro + 0.5 mM DHP (pJF1576) | 99.2 | 99.6 | 99.7 | 99.3 | 96.6 | 96.1 | 92.9 |

**Table 24: Productivity profiles of cell populations cultured in batch fermentation in shake flasks under non-selecting conditions.**

| | **Productivity profile [mg/L]** | **Day after inoculation** | | | | | | |
|---|---|---|---|---|---|---|---|---|
| **#** | **Condition under which stable cell population was selected** | **0** | **3** | **5** | **7** | **10** | **12** | **14** |
| 1 | CD-Fusion + 10 µg/ml Puromycin (pJF1579) | nd | 12.4 | 24.4 | 60.0 | 155.1 | 265.9 | 372.0 |
| 2 | CD-Fusion + 10 µg/ml Puromycin (pJF1576) | nd | 13.6 | 26.6 | 68.9 | 182.0 | 304.9 | 442.2 |
| 3 | CD-Fusion w/o Pro + 0.2 mM DHP (pJF1576) | nd | 18.0 | 44.0 | 142.2 | 435.7 | 669.5 | 779.7 |
| 4 | CD-Fusion w/o Pro + 0.4 mM DHP (pJF1576) | nd | 27.5 | 68.7 | 171.4 | 330.6 | 503.9 | 727.9 |
| 5 | CD-Fusion w/o Pro + 0.5 mM DHP (pJF1576) | nd | 10.3 | 18.2 | 43.4 | 111.7 | 191.4 | 286.8 |

### Example 11

### Optimal concentration of DHP is a function of positioning of P5CS expression cassette

To test the hypothesis that the positioning of P5CS expression cassette may influence the concentration level needed for improved selections employing DHP, two different single selection marker vectors expressing GFP were compared: pJF2055 with P5CS expression cassette (long isoform) downstream of GFP cassette, and pJF2303 with P5CS expression cassette (long isoform) upstream of GFP expression cassette. The long isoform of P5CS was chosen to prevent any uncontrolled influence by inhibition of Ornithine that may be produced at low levels by the cells during cultivation. The vectors pJF2053 and pJF2222, containing Puromycin selection marker expression cassettes down- and upstream of GFP cassette, respectively, were taken as a reference. Table 25 shows viabilities determined on day 22 post-transfection. As evident from Table 25, DHP slowed down the recovery of cell populations selected in proline-free medium, indicative of increased selection pressure. This happened at much lower concentrations for pJF2055 than for pJF2303. In this experiment, stable cell populations were expanded under basic selection conditions (i.e. either standard CD Fusion medium containing Puromycin or proline-free CD Fusion medium with Puromycin added as appropriate, but no additional DHP was added). Table 26 summarizes the results obtained using flow cytometric analysis of cell populations on day 22 post-transfection. As evident from Table 26, addition of DHP into selections in proline-free medium had a beneficial impact on expression performance (#1,#2 vs #4 - #6 & #16 - #19). The MFI of the stable cell population was improved by up to 2.6-fold and up to 2.3-fold for pJF2055 and pJF2303, respectively, as compared to Puromycin selected cultures. Similarly, the frequency of high fluorescing cells was increased by up to 7.4-12.7-fold for pJF2055 and up to 5.8-9.9-fold for pJF2303 as compared to Puromycin selected cultures. Although the magnitude of improvement was similar between pJF2055 and pJF2303, the concentration ranges for DHP were different. Apparently, cultures transfected with pJF2055 were about 10-fold more sensitive to DHP treatment. These results are consistent with the hypothesis that positioning of P5CS expression cassette influences the concentration level needed for improved selections employing DHP. This level may need to be determined individually for each vector setup and expression system. The results also indicate that an optimal selection concentration exists. Further increases in concentration of DHP do not necessarily lead to further increases in expression performance.

**Table 25: Cell viabilities determined post-transfection on the day indicated. Culture #3 & #7 were lost during the experiment due to technical failure.**

| Vector | # | Selection Condition | Viability on day 22 post-transfection [%] |
|---|---|---|---|
| pJF2053 | 1 | CD-Fusion + 10 µg/ml Puromycin | 98.6 |
| pJF2222 | 2 | CD-Fusion + 10 µg/ml Puromycin | 98.7 |
| pJF2055 | 3 | CD-Fusion w/o Pro | lost |
| | 4 | CD-Fusion w/o Pro + 0.02 mM DHP | 99.5 |
| | 5 | CD-Fusion w/o Pro + 0.03 mM DHP | 99.2 |
| | 6 | CD-Fusion w/o Pro + 0.04 mM DHP | 99.2 |
| | 7 | CD-Fusion w/o Pro + 0.05 mM DHP | lost |
| | 8 | CD-Fusion w/o Pro + 0.06 mM DHP | 83.3 |
| | 9 | CD-Fusion w/o Pro + 0.07 mM DHP | 65.3 |
| | 10 | CD-Fusion w/o Pro + 0.08 mM DHP | 36.3 |
| pJF2303 | 11 | CD-Fusion w/o Pro | 99.6 |
| | 12 | CD-Fusion w/o Pro + 0.02 mM DHP | 99.3 |
| | 13 | CD-Fusion w/o Pro + 0.04 mM DHP | 99.6 |
| | 14 | CD-Fusion w/o Pro + 0.06 mM DHP | 99.4 |
| | 15 | CD-Fusion w/o Pro + 0.08 mM DHP | 99.2 |
| | 16 | CD-Fusion w/o Pro + 0.2 mM DHP | 98.4 |
| | 17 | CD-Fusion w/o Pro + 0.3 mM DHP | 98.2 |
| | 18 | CD-Fusion w/o Pro + 0.4 mM DHP | 98.5 |
| | 19 | CD-Fusion w/o Pro + 0.5 mM DHP | 99.1 |

**Table 26: Flow cytometric analysis using Guava easyCyte 8HT Benchtop Flow Cytometer. Yellow Fluorescence Channel was used due to the high expression of GFP. Median Fluorescence Intensity (MFI) as well as frequency of high fluorescent cells were determined. nd = not determined.**

| **Vector** | **#** | **Selection Condition** | **Median Fluorescence Intensity (MFI) (yellow)** | **Frequency of high fluorescing cells [%]** |
|---|---|---|---|---|
| pJF2053 | 1 | CD-Fusion + 10 µg/ml Puromycin | 11308 | 2.5 |
| pJF2222 | 2 | CD-Fusion + 10 µg/ml Puromycin | 11476 | 4.2 |
| pJF2055 | 3 | CD-Fusion w/o Pro | nd | nd |
| | 4 | CD-Fusion w/o Pro + 0.02 mM DHP | 24032 | 17.3 |
| | 5 | CD-Fusion w/o Pro + 0.03 mM DHP | 22607 | 18.5 |
| | 6 | CD-Fusion w/o Pro + 0.04 mM DHP | 29703 | 31.3 |
| | 7 | CD-Fusion w/o Pro + 0.05 mM DHP | nd | nd |
| | 8 | CD-Fusion w/o Pro + 0.06 mM DHP | 15210 | 6.9 |
| | 9 | CD-Fusion w/o Pro + 0.07 mM DHP | 12393 | 8.7 |
| | 10 | CD-Fusion w/o Pro + 0.08 mM DHP | 9617 | 9.3 |
| pJF2303 | 11 | CD-Fusion w/o Pro | 10199 | 2.4 |
| | 12 | CD-Fusion w/o Pro + 0.02 mM DHP | 12080 | 3.3 |
| | 13 | CD-Fusion w/o Pro + 0.04 mM DHP | 15503 | 5.7 |
| | 14 | CD-Fusion w/o Pro + 0.06 mM DHP | 12536 | 3.2 |
| | 15 | CD-Fusion w/o Pro + 0.08 mM DHP | 14603 | 3.7 |
| | 16 | CD-Fusion w/o Pro + 0.2 mM DHP | 19163 | 14.7 |
| | 17 | CD-Fusion w/o Pro + 0.3 mM DHP | 22030 | 17.4 |
| | 18 | CD-Fusion w/o Pro + 0.4 mM DHP | 26155 | 24.5 |
| | 19 | CD-Fusion w/o Pro + 0.5 mM DHP | 23616 | 19.6 |
| | 20 | CHO-K1 parental host cell line | 110 | 0.0 |

### Example 12

A weakened promoter controlling P5CS results in increase in stringency and production yields and can be further combined with DHP
To further explore the hypothesis that the amount of P5CS produced may modulate the optimal DHP concentration window useful for selective growth conditions, the pJF2460 vector was cloned, in which P5CS expression was driven by a weakened SV40 promoter (Benoist & Chambon, Nature. 1981 Mar 26;290(5804):304-10). In pJF2460, P5CS expression was driven by the truncated Delta3SV40E promoter.The vectors pJF2053 and pJF2055 contain a Puromycin selection marker expression cassette as well as a P5CS gene driven by a wild-type SV40 promoter, respectively and were used as a reference. Table 27 shows viabilities determined on day 15 post-transfection. As evident from Table 27, unexpectedly, recovery of cell populations selected in proline-free medium using a truncated SV40 promoter driving expression of P5CS was slowed down as compared to wild-type SV40 promoter, indicative of increased selection pressure, even without adding DHP. Moreover, DHP slowed down the recovery of cell populations selected in proline-free medium, indicative of increased selection pressure. In fact, cultures #6 & #7 failed to fully recover until day 29. In this experiment, stable cell populations were expanded under basic selection conditions (i.e. either standard CD Fusion medium containing Puromycin or proline-free CD Fusion medium, but no additional DHP was added). Table 28 summarizes the results obtained using flow cytometric analysis of cell populations on day 29 post-transfection. As evident from Table 28, addition of DHP into selections in proline-free medium had a beneficial impact on expression performance (#1,#2 vs #3, #4, #5). The MFI of the stable cell population was improved by up to 2.8-fold for pJF2460 as compared to Puromycin selected cultures. Similarly, the frequency of high fluorescing cells was increased by up to 19-fold for pJF2460 as compared to Puromycin selected cultures. Remarkably, the use of a weakened SV40 promoter in pJF2460 plasmid already led to a superior expression performance, even without the use of DHP. This observation is consistent with a truncated, weakened promoter yielding lower amounts of P5CS and consequently influencing the concentration level needed for improved selections employing DHP. This even allows selecting superior stable cell populations in the absence of DHP. At the same time, lower levels of DHP can be used to further augment the effect.

**Table 27: Cell viabilities determined post-transfection on the day indicated.**

| **Vector** | **#** | **Selection Condition** | **Viability on day 15 post-transfection [%]** |
|---|---|---|---|
| pJF2053 | 1 | CD-Fusion + 10 µg/ml Puromycin | 86.6 |
| pJF2055 | 2 | CD-Fusion w/o Pro | 96.3 |
| pJF2460 | 3 | CD-Fusion w/o Pro | 88.2 |
| | 4 | CD-Fusion w/o Pro + 0.01 mM DHP | 73.2 |
| | 5 | CD-Fusion w/o Pro + 0.03 mM DHP | 44.1 |
| | 6 | CD-Fusion w/o Pro + 0.04 mM DHP | 30.6 |
| | 7 | CD-Fusion w/o Pro + 0.05 mM DHP | 22.8 |

**Table 28: Flow cytometric analysis using Guava easyCyte 8HT Benchtop Flow Cytometer. Yellow Fluorescence Channel was used due to the high expression of GFP. Median Fluorescence Intensity (MFI) as well as frequency of high fluorescent cells were determined.**

| **Vector** | **#** | **Selection Condition** | **Median Fluorescence Intensity (MFI) (yellow)** | **Frequency of high fluorescing cells [%]** |
|---|---|---|---|---|
| pJF2053 | 1 | CD-Fusion + 10 µg/ml Puromycin | 11502 | 2.3 |
| pJF2055 | 2 | CD-Fusion w/o Pro | 13835 | 2.7 |
| pJF2460 | 3 | CD-Fusion w/o Pro | 24986 | 22.2 |
| | 4 | CD-Fusion w/o Pro + 0.01 mM DHP | 19951 | 15.3 |
| | 5 | CD-Fusion w/o Pro + 0.03 mM DHP | 31838 | 43.6 |
| | 12 | CHO-K1 parental host cell line | 4 | 0.0 |

### Example 13

### Proline-dependent viability of CHO cells

CHO-S cells (Thermo Fisher Scientific, A1155701) were passaged according to the manufacturer's recommendations at 37 °C and 5% CO₂ in CD-CHO medium (Thermo Fisher Scientific , 10743029) containing 8 mM GlutaMAX. Cells in logarithmic growth phase were diluted to 2e5 viable cells per ml in different media: standard CD-CHO medium, CD-CHO medium without proline and without ornithine (proline-free CD-CHO medium) and proline-free CD-CHO medium into which different amounts of proline had been added back. Viable cell densities and viabilities were recorded daily.

| | **Viability** | | | | |
|---|---|---|---|---|---|
| | Day 0 | Day 1 | Day 2 | Day 3 | Day 6 |
| CD CHO+ Glutamax 8mM | 99,6 | 98,5 | 97,3 | 95,9 | 99,4 |
| Proline-free Medium without L-Prolin | 99,6 | 99,0 | 95,4 | 65,9 | 21,3 |
| Proline-free Medium + 0,25mM L-Prolin | 99,6 | 95,6 | 98,1 | 99,6 | 98,2 |
| Proline-free Medium + 0,5mM L-Prolin | 99,6 | 98,2 | 99,7 | 99,5 | 99,9 |
| Proline-free Medium + 1,0mM L-Prolin | 99,6 | 100,0 | 99,7 | 99,2 | 99,5 |
| Proline-free Medium + 2,5mM L-Prolin | 99,6 | 99,3 | 97,5 | 99,8 | 99,7 |

| L-Prolin Fa. Sigma, #P5607 | | | | | |
|---|---|---|---|---|---|
| | **Viable Cell Conc.** | | | | |
| | Day 0 | Day 1 | Day 2 | Day 3 | Day 6 |
| CD CHO+ Glutamax 8mM | 2,00E+05 | 2,9E+05 | 7,9E+05 | 2,0E+06 | 8,9E+06 |
| Proline-free Medium without L-Prolin | 2,00E+05 | 2,2E+05 | 1,9E+05 | 1,5E+05 | 3,5E+04 |
| Proline-free Medium + 0,25mM L-Prolin | 2,00E+05 | 3,5E+05 | 5,7E+05 | 9,1E+05 | 1,3E+06 |
| Proline-free Medium + 0,5mM L-Prolin | 2,00E+05 | 3,7E+05 | 6,3E+05 | 1,2E+06 | 3,8E+06 |
| Proline-free Medium + 1,0mM L-Prolin | 2,00E+05 | 3,6E+05 | 5,6E+05 | 1,1E+06 | 6,6E+06 |
| Proline-free Medium + 2,5mM L-Prolin | 2,00E+05 | 3,3E+05 | 4,9E+05 | 1,1E+06 | 6,3E+06 |

## Claims

1. Use of 3,4-dehydroproline (DHP) and/or at least one of its salts in a metabolic selection system, wherein the metabolic selective marker is a functional pyrroline-5-carboxylate synthase (P5CS).

2. A method for culturing and screening eukaryotic cells for the expression of high levels of one or more protein(s) of interest (POI), the method comprising
a. introducing one or more polynucleotide(s) into a eukaryotic cell, wherein the one or more polynucleotide(s) encode(s) a functional P5CS and at least one POI,
b. culturing the cell in an essentially proline-free cell culture medium, and
c. culturing the cell in the presence of DHP and/or at least one of its salts.

3. A polynucleotide comprising a nucleic acid sequence encoding a functional P5CS and at least one POI, wherein the expression of the functional P5CS and the expression of at least one POI are under the control of different promoters, and wherein the promoter controlling the expression of the functional P5CS is a weak promoter, such as a SV40 promoter, a truncated SV40 promoter or a Delta3SV40E promoter.

4. A polynucleotide comprising a nucleic acid sequence encoding a functional P5CS and at least one POI, wherein the expression of the functional P5CS and the expression of at least one POI are under the control of different promoters, and wherein the promoter controlling said functional P5CS is weaker than the promoter controlling said at least one POI.

5. A polynucleotide according to any of the claims 3 to 4, wherein the polynucleotide comprises a nucleic acid sequence as set forth in SEQ ID NO:2, SEQ ID NO:3, SEQ ID NO:5 and/or SEQ ID NO:6.

6. A polynucleotide according to any of the claims 3 to 5, wherein the polynucleotide further encodes for an additional selective marker protein, such as a further metabolic selective marker or an antibiotic selective marker.

7. A polynucleotide according to any of the claims 3 to 6, wherein the polynucleotide further encodes for a reporter protein, such as a fluorescent protein.

8. A polynucleotide according to any of the claims 3 to 7, wherein the polynucleotide further comprises one or more ubiquitous chromatin opening element(s) (UCOE).

9. A method for culturing and screening eukaryotic cells for the expression of high levels of at least one POI, the method comprising
a. introducing a polynucleotide according to any of the claims 3 to 8 into a eukaryotic cell, and
b. culturing the cell in an essentially proline-free cell culture medium.

10. A method according to claim 9, wherein the method further comprises culturing the cell in the presence of DHP and/or at least one of its salts.

11. An essentially proline-free cell culture medium comprising DHP and/or at least one of its salts for culturing and screening eukaryotic cells.

12. An essentially proline-free cell culture medium according to claim 11, wherein said medium is based on a medium for culturing CHO cells, such as EX-CELL CD CHO fusion medium.

13. The essentially proline-free cell culture medium according to any of the claims 11 to 12, wherein the concentration of DHP and/or at least one of its salts is between 0.001 mM and 100 mM.

14. A cell culture medium according to any of the claims 11 to 13, wherein the essentially proline-free cell culture medium is essentially free of ornithine.

15. A eukaryotic cell, wherein at least one polynucleotide according to claims 3 to claim 8 has been introduced.

16. The eukaryotic cell according to claim 15, wherein the eukaryotic cell is a Chinese Hamster Ovary (CHO) cell, such as a CHO-K1, CHO-S, CHO-K1SV, CHO Freestyle, CHOEBNALT85, CHOS-XE, CHO-DG44, CHO-DXB11 or CHO-3E7 cell.

17. The eukaryotic cell according to any of the claims 15 to 16, wherein the expression of functional P5CS renders the cell proline prototroph.
